(19) Europäisches Patentamt / European Patent Office / Office européen des brevets

(11) **EP 3 290 087 B1**

(12) **EUROPEAN PATENT SPECIFICATION**

(45) Date of publication and mention
of the grant of the patent:
**14.10.2020 Bulletin 2020/42**

(51) Int Cl.:
*A61K 8/73* *(2006.01)*      *A61Q 13/00* *(2006.01)*
*A61K 8/04* *(2006.01)*      *C11B 9/00* *(2006.01)*

(21) Application number: **17188696.3**

(22) Date of filing: **31.08.2017**

(54) **SPRAY COMPOSITIONS, SPRAYABLE PRODUCTS, AND METHODS OF TREATING A SURFACE WITH THE SAME**

SPRÜHZUSAMMENSETZUNGEN, SPRÜHBARE PRODUKTE UND VERFAHREN ZUR BEHANDLUNG EINER OBERFLÄCHE DAMIT

COMPOSITIONS DE PULVÉRISATION, PRODUITS PULVÉRISABLES ET PROCÉDÉS DE TRAITEMENT D'UNE SURFACE AVEC CEUX-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.09.2016 US 201662383646 P**

(43) Date of publication of application:
**07.03.2018 Bulletin 2018/10**

(73) Proprietor: **The Procter & Gamble Company
Cincinnati, OH 45202 (US)**

(72) Inventors:
• **HORENZIAK, Steven Anthony
Cincinnati, Ohio 45202 (US)**
• **STURGIS, David Arhur
Cincinnati, Ohio 45202 (US)**
• **LI, Jianjun Justin
Cincinnati, Ohio 45202 (US)**
• **FLICKINGER, Marc Adam
Cincinnati, Ohio 45202 (US)**
• **HUTCHINS, Virginia Tzung-Hwei
Cincinnati, Ohio 45202 (US)**
• **DIERSING, Steven Louis
Cincinnati, Ohio 45202 (US)**
• **WUJEK, Steven Michael
Cincinnati, Ohio 45202 (US)**

(74) Representative: **P&G Patent Belgium UK
N.V. Procter & Gamble Services Company S.A.
Temselaan 100
1853 Strombeek-Bever (BE)**

(56) References cited:
**WO-A1-2014/151555      WO-A2-2008/104960
US-A1- 2008 213 204**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

This application generally relates to spray compositions, products comprising spray compositions, and methods of treating a surface with the spray compositions, and, particularly, relates to spray compositions comprising a perfume-cyclodextrin complex.

BACKGROUND OF THE INVENTION

Perfume compositions are utilized in spray compositions to help make products more delightful to consumers. This is especially true for perfume compositions and complexes that can provide a desired and long-lasting fragrance or scent each time the composition is applied or used. However, current perfume compositions are not optimized for release from a cyclodextrin complex and some components can remain within the complex and unexpressed. As such, there is a need for a perfume composition which is optimized for release from a cyclodextrin and cyclodextrin perfume complexes made from such optimized perfumes.

SUMMARY OF THE INVENTION

Combinations:

A. A spray composition comprising:

about 0.01 wt.% to about 3.0 wt.% perfume-cyclodextrin complex, by weight of the overall composition, the perfume-cyclodextrin complex comprising:
perfume raw materials, wherein 50% or more, by weight of the perfume raw materials have: a cyclodextrin complex stability constant (log k) of less than about 3.0, a ClogP of about 2.5 or less; and a weight average molecular weight of about 200 Daltons or less; and cyclodextrin;
about 30 wt.% to about 80 wt.% solvent, by weight of the overall composition;
about 0.01 wt.% to about 3.0 wt. % free perfume composition, by weight of the overall composition; and
less than 10 wt. % water, by weight of the overall composition.

B. The spray composition according to Paragraph A, wherein the solvent is selected from the group consisting of: monohydric alcohols, polyhydric alcohols, hydrocarbons, and combinations thereof.
C. The spray composition according to Paragraph A or Paragraph B further comprising a malodor counteractant.
D. The spray composition according to any of Paragraphs A through C, wherein the cyclodextrin complex stability constant (log k) is from about -2.0 to about 2.5.
E. The spray composition according to any of Paragraphs A through D, wherein about 20% to about 100%, by weight of the perfume-cyclodextrin complex, of the perfume raw materials have: a complex stability constant of about 3.0 or less, a ClogP of about 2.5 or less; and a weight average molecular weight of about 200 Daltons or less.
F. The spray composition according to any of Paragraphs A through E, wherein the perfume raw materials have a weight average molecular weight of about 180 Daltons or less.
G. A sprayable product comprising:

a spray dispenser;
a spray composition comprising:
a perfume-cyclodextrin complex, by weight of the total composition, the perfume-cyclodextrin complex comprising:

perfume raw materials, wherein 50% or more, by weight of the perfume raw materials have: a cyclodextrin complex stability constant (log k) of less than about 3.0, a ClogP of about 2.5 or less; and a weight average molecular weight of about 200 Daltons or less; and
cyclodextrin;
a solvent; and
less than 10 wt. % water, by weight of the total composition; and
a propellant.

H. The sprayable according to Paragraph G, wherein the propellant is selected from the group consisting of: butane, propane, or isobutene as propellant, and combinations thereof.

I. The sprayable product according to Paragraph G or Paragraph H, wherein the propellant is selected from the group consisting of: nitrogen, carbon dioxide, air, and combinations thereof.

J. The sprayable product according to any of Paragraphs G-I, wherein the weight ratio of spray composition to propellant is about 30:70 to 70:30.

K. The sprayable product according to any of Paragraphs G-J, wherein the perfume-cyclodextrin complex is present at a level of about 0.01 wt.% to about 3.0 wt.%, by weight of the total composition.

L. The sprayable product according to any of Paragraphs G-K further comprising about 0.01 wt.% to about 2.0 wt. % free perfume composition, by weight of the total composition.

M. The sprayable product according to any of Paragraphs G-L, wherein the solvent is present at a level of about 20 wt.% to about 80 wt.% by weight of the spray composition.

N. The sprayable product according to any of Paragraphs G-M, wherein the solvent is selected from the group consisting of: monohydric alcohols, polyhydric alcohols, hydrocarbons, and combinations thereof.

O. A method of treating a surface, the method comprising the steps of:

spraying a surface with a spray composition, the spray composition comprising:

a perfume-cyclodextrin complex, by weight of the total composition, the perfume-cyclodextrin complex comprising:

perfume raw materials, wherein 50% or more, by weight of the perfume raw materials have: a cyclodextrin complex stability constant (log k) of less than about 3.0, a ClogP of about 2.5 or less; and a weight average molecular weight of about 200 Daltons or less; and
cyclodextrin;
a solvent; and
less than 10 wt. % water, by weight of the total composition; and
exposing the surface to water.

P. The spray composition of any of the preceding Paragraphs A-O, wherein the cyclodextrin complex stability constant (log k) is from about -2.0 to about 2.5.

Q. The spray composition of any of the preceding Paragraphs A-P, wherein the perfume raw materials are selected from the group consisting of: eugenyl formate; benzaldehyde; 2-hexen-1-yl acetate; alpha-methyl cinnamaldehyde; methyl phenylacetate; viridine; ethyl 2-phenylacetate; methyl hydrocinnamate; methyl cinnamate; 2-Phenylethyl acetate; cinnamyl acetate; lilac acetaldehyde; 4-(p-Methoxyphenyl)-2-butanone; anethole; gamma-Octalactone; 3-phenyl propionaldehyde; cinnamic alcohol; cinnamic aldehyde; phenethyl formate; 3-phenyl propyl formate; isobutyl furylpropionate; styryl acetate; geranyl formate; 3-Hepten-1-ol; citronellol; trans-Geraniol; nerol; neral; melon heptenal; propyl mercaptan; 2-Propionylpyrrole; 5,6-Dimethyl-1-(1-methylethenyl)bicyclohept-5-ene-2-methanol; hydratopic alcohol; 3,4-Dimethoxyacetophenone; safranal; 2-Hydroxyacetophenone; cis-carveol, ocean propanal; Isosafrol; Indole; 2-Methylbenzothiazole; Ethyl vanillin; Vanillin; Methyl p-anisate; Benzyl propionate; 3-phenyl propyl acetate; phenyl acetaldehyde; p-Hydroxybenzaldehyde; para-anisaldehyde; Isoamyl acetate; Ethyl 3-methylthiopropionate; Methyl anthranilate; 1,2-Cyclopentanedione, 3-ethyl-; Syringaldehyde; furfuryl thioacetate; blackberry thiophenone; p-Cresyl acetate; linalool oxide (pyranoid); Geranial; Parmanyl; Sorbinaldehyde; Pentyl 2-furyl ketone; m-Guaiacol; alpha-Methylcinnamic alcohol; Ethyl cyclohex-3-enecarboxylate; 2,4-Hexadienyl acetate; 4-Hydroxy-3-methylbenzaldehyde; Furan, 3-methyl-2-(3-methyl-2-butenyl)-; n-Pentyl acetoacetate; Ethyl 2-hexenoate; 2-Ethyl-4-methylthiazole; tropical thiazole; Trifernal; Coumarone; 2,4-Hexadienyl propionate; Cyclopentyl mercaptan; 2-Methyl-2-butanethiol; trans-2-Methyl-2-pentenoic acid; 2-Hexyl-1,3-dioxolane; cis-3-Hepten-1-ol; 3-Hexenyl acetate; Trans,trans-2,4-Hexadien; methyl trans-cinnamate 99%; 4-Methyl-5-vinylthiazole; 2-Propylthiazole; (S),(-)-Perillaaldehyde; 2-(1-Methylpropyl)thiazole; (+)-p-menth-1-en-9-OL 97% (mixture of isomers); Isobutyl thiazole; trans-2-Heptenal; (1S)-(-)-cis-Verbenol; Anapear; alpha-Campholenic alcohol; Ethyl 2-mercaptopropionate; 2-Methylphenethyl alcohol; Methyl 4-phenylbutyrate; Allyl crotonate; Allyl butyrate; Benzyl lactate; Vanillin isobutyrate; perillaldehyde; Neryl Formate; Allyl methyl disulfide; Methyl propyl disulfide; 2-Cyclopenten-1-one, 2-hydroxy-3,4-dimethyl-; 3-Ethyl-2-hydroxy-2-cyclopenten-1-one; 2-Octenol-1; Tetrahydrofurfuryl butyrate; Allo-ocimenol; 7-Octene-1,6-diol, 3,7-dimethyl-; 3-Ethoxybenzaldehyde; 2-Ethylbenzaldehyde; 2-hexen-1-ol; Phenoxyethyl propionate; Nerolione; 7-Methylcoumarin; Butylacrolein; 2-Hexen-1-yl acetate; Ethyl phenoxyacetate; Ethyl trans-3-hexenoate; N-Acetyl methyl anthranilate; Ethyl trans-2-hexenoate; Vertoliff; (2E,6Z)-Nona-2,6-dien-1-ol; Ocimenol; 2,6-Dimethyl-1,5,7-octatrienol-3; p-Menth-1-ene-9-al; 2,4-Octadien-1-al; Propyl anthranilate; 2-Pentanoylfuran; 4-Ethyl-2-methylthiazole; Jasmolactone; cis-3-Hexenyl formate; 1-Octenol-3; 4,5-Dimethylthiazole; 4,4-Dimethyl-5-isopropyl-1,3-dioxolane; 1-Hexen-3-yl acetate; Furfuryl valerate; 2,6-Dimethyl-6-hepten-1-ol; cis-3-Hexenyl acetate; trans-3-Hexenyl acetate; 5-Ethyl-2-thiophenecarbaldehyde; 2-Phenyl-1(2)propenyl-1 ester; 3-Cyclohexene-1-ethanol, 4-methyl-beta-methylene-, (R)-; Furfuryl hexanoate; 3-methoxy cinnamaldehyde; 3-Acetyl-5-butyldihydro-2(3H)-furanone; Pyrazine, 3-butyl-2,5-dimethyl-; Methyl Heptenone; 2,5-Dimethylthiazole; (E)-anethol; Phenylethyl oxy-acetaldehyde; 3-Ethyl-2-hydroxy-4-methylcyclopent-2-en-1-one; (E,E)-2,4-heptadien-1-al; Cinnamic aldehyde dimethyl acetal; Campholene aldehyde; cis-4-Hexenal; 2-Hepten-4-one; 2-Octen-4-one; Verbenol; 4-Ethylbenzaldehyde; Piperitol; piperitenone; Isocoumarin; Lepidine; ethyl maltol; Butyroin; Hinokitiol; Pyrazine, 2-butyl-3,5-dimethyl-; cis-3, cis-6-nonadienol; trans-2-Hexenyl formate; Ethyl 2-methyl-4-pentenoate; 1-(4-Methylphenyl)ethanol; Perillyl alcohol; Cumic alcohol; citral; Benzyl acetoacetate; p-Methylhydrocinnamic aldehyde; 2,4-Dimethylthiazole; Acetaldehyde phenyl ethyl acetal; Canthoxal; Ethyl 3-mercaptopropionate; Raspberry ketone; 2-Methylthiophene; 3,6-Octadienal, 3,7-dimethyl-; 2,4-Octadienal; Cinnamaldehyde ethylene glycol acetal; trans-3, cis-6-nonadienol; 2-Heptenal, (2Z)-; Methyl furfuryl disulfide; o-Acetylanisole; Lavandulol; 3-Methylacetophenone; p-Tolyl alcohol; Furfuryl thiopropionate; 2-Mercaptomethylpyrazine; 2,4-Heptadienal; cis-iso-Eugenol; S-Ethyl benzothioate; trans-Isoeugenol; Methyl 2-nitrobenzoate; Methyl o-methoxybenzoate; Guaiacyl acetate; 3-Methylthiophene; cis-4-Hepten-1-ol; beta-Phenoxy ethyl acetate; cis-3-Hexenyl lactate; meta-tolyl aldehyde; 4-(2-Furyl)-3-buten-2-one; Dimethyl disulfide; Cyclopentyl isobutyrate; Phenylacetaldehyde diethyl acetal; tetrahydrofurfuryl propionate; 2,5-Dimethylthiophene; Ethyl 2-methoxybenzyl ether; p-Methoxy-alpha-methyl cinnamaldehyde; Geranyl oxyacetaldehyde; Ethyl (p-tolyloxy)acetate; Trans-2-Hexenal; cis-4-Heptenal; 3-Mercapto-2-pentanone; 3,5,6-Trimethyl-3-cyclohexene-1-carbaldehyde; Floralol; 2,4-Dimethyl-3-cyclohexene-1-methanol; trans-2-Hexenal diethyl acetal; 3,6-ivy carbaldehyde; p-Methyl phenoxy acetaldehyde; (Z)-3-hexen-1-al; Dimethyl cyclohexene carboxaldehyde; 2,4-Dimethyl-3-Cyclohexene-1-carboxaldehyde; cis-3-Hexenyl pyruvate; 3,5-ivy carbaldehyde; delta-Octalactone; Methyl benzyl disulfide; 1-Phenylbutan-2-ol; Ethyl 2-methylbutyrate; Methyl mercaptan; Allyl anthranilate; Allyl tiglate; Ethanethiol; dimethyl sulfide 2-Propanethiol; (-)-Citronellol; Anisyl propionate; tert-Butyl mercaptan; 2,4-Pentadienal; 3,6-Nonadien-1-ol; Benzaldehyde diethyl acetal; 2-Thienyl mercaptan; 4-(p-Tolyl)-2-butanone; Isoeugenyl formate; 2,6-Nonadien-1-ol; 2-Methoxy-4-vinylphenol; p-Menth-8-en-3-ol; filbert heptenone; Gardamide; Dimethyl anthranilate; Allyl mercaptan; Ethyl anthranilate; cinnamon acrolein; Vanillin acetate; Isopulegol; Salicylaldehyde; Guaiacol; Hydratropaldehyde dimethyl acetal; Coumarin (Z)-2-hexen-1-ol; (E)-2-hexen-1-ol; cis-3-Hexen-1-ol; Methyl isoeugenol; Isoeugenyl acetate; 2-phenyl propionaldehyde; 1-Phenyl-1-propanol; Methyl benzoate; Ethyl benzoate; Citronitrile; Styrallyl acetate; Butanoic acid, 2-methyl-, 2-hexenyl ester, (E)-; Vanitrope; 2-Methylindole; Eugenol; and a combination thereof.

R. The spray composition of any of the preceding Paragraphs A-O, wherein the perfume raw materials are selected from the group consisting of: beta gamma hexanol; cis 3 hexenyl acetate; ethyl-2-methyl butyrate; amyl-acetate; vanillin; anethole; methyl isoeugenol; guaiacol; floralol; 2,6-nonadien-1-ol; coumarin; and a combination thereof.

S. The spray composition of any of the preceding Paragraphs A-O, wherein the perfume raw materials comprise dimethyl anthranilate; iso-eugenyl acetate; canthoxal; 3,6-nonadien-1-ol, triplal; or a combination thereof.

T. The spray composition of any of the preceding Paragraphs A-O, wherein the perfume raw materials comprise ethyl-2-methyl butyrate; beta gamma hexanol; iso amyl acetate; amyl acetate; cis-3-hexenyl acetate; gamma-octalactone; ethyl vanillin; vanillin; benzaldehyde; or a combination thereof.

U. The spray composition of any of the preceding Paragraphs A-T, wherein the 10% or more of the perfume raw materials also have an Odor Detection Threshold of about 7 or more - log molar concentration.

V. The spray composition of any of the preceding Paragraphs A-U, wherein 10% or more of the perfume raw materials also have an Odor Detection Threshold of about 7 to about 11.5 -log molar concentration.

W. The spray composition of any of the preceding Paragraphs A-V, wherein about 50% to about 100%, by weight of the perfume, of the perfume raw materials have: a cyclodextrin binding coefficient of less than about 3.0, a ClogP of about 2.5 or less; and a weight average molecular weight of about 200 Daltons or less.

X. The spray composition of any of the preceding Paragraphs A-W, wherein about 50% to about 100%, by weight of the perfume-cyclodextrin complex, of the perfume raw materials have: a cyclodextrin binding coefficient of about -2.0 to less than about 3, a ClogP of about 2.5 or less; and a weight average molecular weight of about 200 Daltons or less.

Y. The spray composition of any of the preceding Paragraphs A-X, wherein the perfume raw materials have a cyclodextrin binding coefficient of about -1.5 to about 2.5.

Z. The spray composition of any of the preceding Paragraphs A-O, wherein the perfume raw materials have a ClogP of about 2.0 or less.

AA. The spray composition of any of the preceding Paragraphs A-O, wherein the perfume raw materials have a weight average molecular weight of about 180 Daltons or less.

BRIEF DESCRIPTION OF THE DRAWINGS

FIG. 1 is a side-by-side comparison of the cyclodextrin complex stability constant (BCD binding strength) of a perfume composition before and after optimization for release from a cyclodextrin complex;

FIG. 2 is a side-by-side comparison of the cyclodextrin complex stability constant over LogP of a perfume composition before and after optimization for release from a cyclodextrin complex;

FIG. 3 is a graph showing the percentage of perfume complexed with a beta cyclodextrin that is released when measured in accordance with the In Vitro Perfume Release Method; and

FIG. 4 is a graph showing the average scent intensity at each assessment time point, where 1 is at application, 2 is during the day, and 3 is at the end of the day.

DETAILED DESCRIPTION OF THE INVENTION

"Cyclodextrin complex stability constant" (log K) refers to the ability of a perfume raw material to bind to a cyclodextrin. The complex stability constant of a multitude of materials with respect to various cyclodextrins as measured by the calorimetry technique can be found in the literature, for example, Rekharsky and Inoue (1998), Complexation Thermodynamics of Cyclodextrins, Chemical Review, 98, 1875-1917. In addition, for reference, a list of perfume raw materials and their estimated complex stability constants is included in a table below.

"ClogP" refers to calculated logP values, which is a measure of a compound's hydrophilicity, wherein logP is the octanol water partitioning coefficient as computed by the Consensus algorithm implemented in ACD/Percepta version 14.02 by Advanced Chemistry Development, Inc. (ACD/Labs, Toronto, Canada).

"Odor Detection Threshold" refers to the lowest concentration in the air of a certain odor compound that is perceivable to the human sense of smell. The Odor detection Threshold of a multitude of materials can be found in van Gemert, L.J.; Odour Thresholds (Compilations of Odour Threshold Values in Air, Water and Other Media; Oliemans Punter & Partners; The Netherlands, 2011. It is in units of-log molar concentration. In this context, human odor detection thresholds (ODTs) are expressed as olfactory power, or p.ol (the negative log of the molar concentration of the odorant in air at which a human first detects the presence of the odorant). These values can be directly transposed to other commonly used units such as ppm (volume) and ppb (volume): thresholds of 1ppm and 1ppb are equivalent to p.ol = 6 and p.ol = 9, respectively. Odor Detection Threshold can be measured, for example, by the method in International Publication Number WO 2006/138726.

"Cyclodextrin complex" refers to a complex of cyclodextrin and perfume.

"Molecular weight," unless otherwise designated, refers to the weight average molecular weight which can be calculated by using the sum of the molecular weights of the elements in a molecule. These can be found, for example, in Atomic Weights of the Elements, Weiser, 2005.

"Room temperature as used herein refers to about 20°C.

Many consumers enjoy a good scent in a consumer product. Scent can be delivered through a multitude of means, like direct addition of a scent to a product or through the use of a scent delivery agent. Scent delivery agents can enhance and/or change the delivery of the scent. For example, some delivery agents can encapsulate a so that it can be released upon a triggering event. Other delivery agents can help a deposit onto a target surface so that the perfume is more easily detected by the consumer.

Perfume compositions are usually not a single component, but made up of multiple perfume raw materials which combined give the overall scent of the perfume. Each of the perfume raw materials has its own characteristic and its own chemical properties, like molecular weight, cLogP, etc. These properties can influence where and how long a scent can be detected. Some of these properties are how perfume raw materials are divided into top, middle, and base notes.

Previously, when using a perfume composition in combination with a delivery agent, like complexing a perfume composition with a cyclodextrin, it was believed that most of the perfume composition was released from the delivery agent upon the triggering event. For cyclodextrins, the triggering event is usually the introduction of moisture. However, it was recently discovered that only about 4%, of a complexed perfume composition, was being released from a cyclodextrin upon exposure to moisture. As such, most of the perfume composition was remaining within the cyclodextrin and was not noticeable to the consumer as desired. This means there is significant room for improvement in the efficacy of cyclodextrin and perfume complexes.

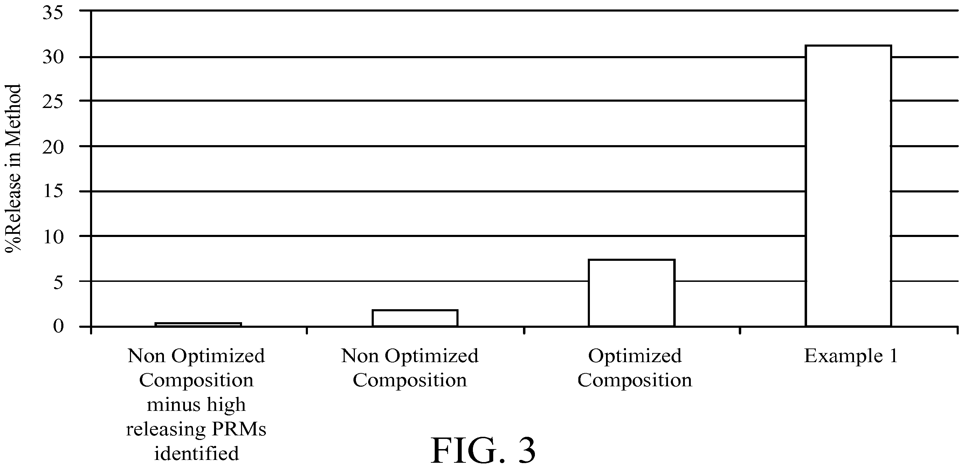

An understanding of what is and what is not releasing from a cyclodextrin could help to improve the efficacy of the perfume cyclodextrin complex. Since less than 5% of the perfume compositions used in a cyclodextrin complex were efficiently releasing from the cyclodextrin complex (see FIG. 3, Non Optimized Composition), the perfume raw materials that were being release from the cyclodextrins were identified to determine if there were characteristics common among them which could be used to help develop a perfume composition for optimized released from a cyclodextrin.

With water being the key releasing agent, it was found that perfume raw materials with more affinity with water (lower log P) had better release from the cyclodextrin complex. Perfume raw materials with a lower cyclodextrin complex stability constant (log k) also had better release from a cyclodextrin complex. In addition, a lower molecular weight, which may correlate with a lower cyclodextrin complex stability constant, also correlates with a batter release. To demonstrate these characteristics as impacting the release from the cyclodextrin composition, new perfume compositions were created. One composition removed these higher releasing perfume raw materials from the original low release composition as a negative control check (see FIG. 3, Non Optimized Composition minus high releasing PRM's identified vs. Non Optimized Composition). In release testing, the Non Optimized Composition minus the high releasing PRM's had less than one third of the release of the original Non Optimized Composition (see FIG. 3).

An optimized composition was then made which utilized about 70%, by weight of the perfume composition, of perfume raw materials with a logP, stability constant, and weight average molecular weight believed to help with perfume release from a cyclodextrin complex. This perfume, Optimized Composition from FIG. 3, had 4 times the release of the original composition (Non Optimized Composition). Another perfume composition was made with 100% of the perfume composition matching these physical property characteristics (Example 1). This perfume composition had over 15 times the release of the Non Optimized Composition.

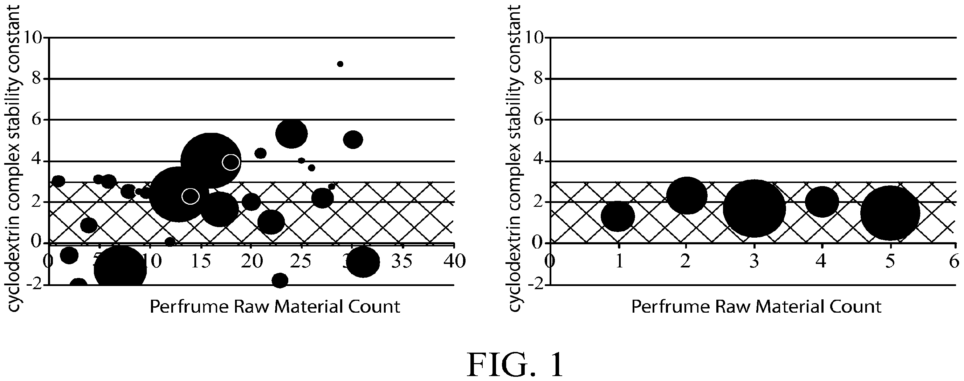

As noted above, one of the characteristics of a perfume raw material that can impact its release from a cyclodextrin is its complex stability constant. This signifies how strongly the perfume raw material binds with the cyclodextrin. While a minimum complex stability constant allows for a perfume raw material to bind and stay bound, at some point the affinity of the perfume raw material for the cyclodextrin can become so strong that it becomes difficult to release. It is believed that a complex stability constant of more than 3 can interfere with the release of the perfume raw material upon a triggering event. This is not to say that perfume raw materials with a complex stability constant above a 3 cannot be used, just that the ability to release such materials should be taken into consideration during perfume design. For example, Fig. 1 shows the binding complex of perfume raw materials in a perfume composition. The graph on the left shows the make-up of a more typical perfume, while the graph on the right shows a perfume composition after optimization for release from a cyclodextrin. The optimized formula showed an improvement of more than 15 times over Non Optimized Perfume A.

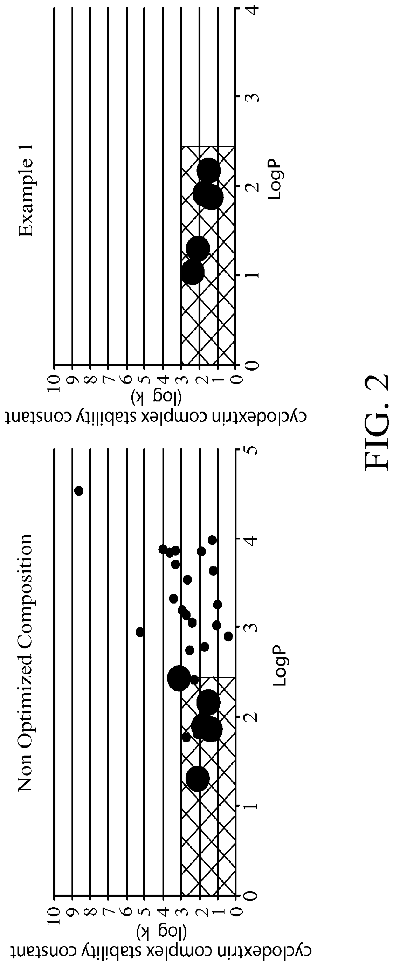

Another property of a perfume raw material which can impact its ability to release from a cyclodextrin is its ClogP. ClogP is the calculation of the logP value of a compound, which is the logarithm of its partition coefficient between n-octanol and water ($C_{octanol}/C_{water}$). Thus, logP, or if calculated cLogP, is a measure of a perfume raw material's hydrophilicity. High logP values correspond to low hydrophilicities. It is believed that a low logP, i.e. higher affinity for water, can positively impact the release of a perfume raw material from a cyclodextrin upon appropriate contact with moisture. For example, Fig. 2 shows the binding complex of perfume raw materials in a perfume and the ClogP. The graph on the left shows the make-up of a more typical perfume, while the graph on the right shows a perfume composition after optimization for release from a cyclodextrin. The optimized formula showed an improvement of 15 times over the Non Optimized Composition. For this application, it is believed a ClogP value of about 2.5 or less is optimal for release from a cyclodextrin complex.

A third property that can impact the release of a perfume raw material from a cyclodextrin is its weight average molecular weight. It is believed that perfume raw materials which are smaller in size will have less binding points to a cyclodextrin and thus more easily released. Ideally, a perfume raw material for optimal release will have a weight average molecular weight of about 200 Daltons or less.

A fourth property that can impact the need for efficacy is the odor detection threshold. Odor detection threshold is the minimum level at which a perfume raw material can be detected by the average human nose. For a perfume raw material with a low odor detection threshold, less of the perfume raw material needs to be released from a cyclodextrin in order for the perfume raw material to be noticed. This feature can allow for the use of perfume raw materials which would otherwise be seen as too difficult to release en masse from a cyclodextrin. Optimally, the odor detection threshold of a perfume raw material is about 7 -log molar concentration or more.

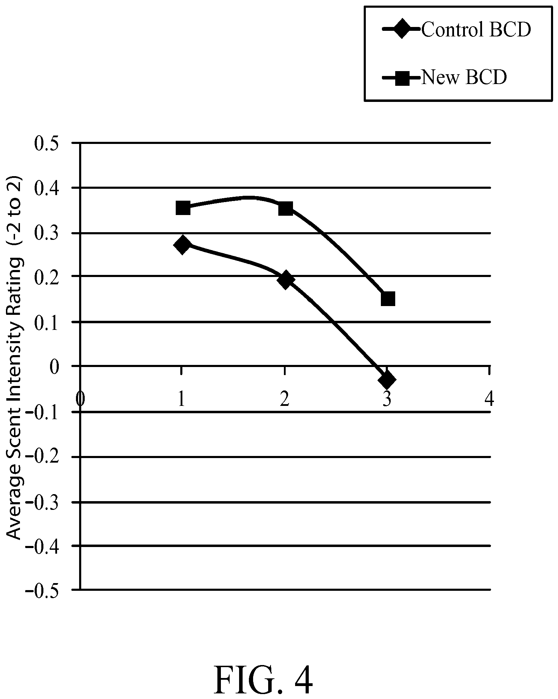

To determine whether the release enhancement was noticeable to consumers, an optimized cyclodextrin perfume complex was tested against an in market complex with less than 5% release. The products were given to over 90 consumers each to wear every day for 2 weeks. After the 2 weeks they were asked to rate the intensity of the on a scale of -2 (much too weak) to 2 (much too strong). They rated the product they wore at application, during the day, and at the end of the day. Fig 4 shows on average those who wore the product with the optimized cyclodextrin reported a higher intensity at each time point evaluated.

Cyclodextrin Complexing Material

A cyclodextrin complexing material may be used for substantially "hiding" a perfume composition until a triggering mechanism has occurred, such as, for example, perspiration, urination, or menstruation, to "release" the perfume composition. As used herein, the term "cyclodextrin" includes any of the known cyclodextrins such as unsubstituted cyclodextrins containing from about six to about twelve glucose units, especially alpha-cyclodextrin, beta-cyclodextrin, gamma-cyclodextrin and/or their derivatives and/or mixtures thereof. For example, cyclodextrins may be selected from the group consisting of beta-cyclodextrin, hydroxypropyl alpha-cyclodextrin, hydroxypropyl beta-cyclodextrin, methylated-alpha-cyclodextrin, methylated-beta-cyclodextrin, and mixtures thereof. Cyclodextrins may be included within a product from at least about 0.1%, from at least about 1%, from at least about 2%, or from at least about 3%; to about 25%, to about 20%, to about 15% or to about 10%, by weight of the composition or article component.

Cyclodextrin particles and cyclodextrin complexes comprising a perfume composition can be formed by various methods. For example, a solvent (e.g., water), unloaded cyclodextrin particles, and a perfume composition can be placed into a container and then mixed for a period of time to permit loading of perfume raw materials into "cavities" of cyclodextrin molecules. The mixture may or may not be processed further; e.g., processed through a colloid mill and/or homogenizer. The solvent is then substantially removed, like by drying, from the resulting mixture or slurry to yield cyclodextrin complex particles. Different manufacturing techniques may however impart different particle/complex characterizations, which may or may not be desirable in the product.

The particles and/or complexes can have a low level of moisture prior to their inclusion into a product. For example, some may have a moisture level of less than about 20% by weight of the particles, less than about 10% by weight of the particles, or even less than about 6% by weight of the particles, prior to the inclusion of the volume of particles into a composition. Other moisture levels may also be suitable.

Spray drying a slurry or mixture of cyclodextrin-perfume complexes is one manufacturing technique capable of producing the cyclodextrin particles and cyclodextrin complexes having the above-noted, low moisture levels. Table I below provides a comparison of spray dried cyclodextrin complexes versus complexes formed via an extruder process (kneading).

Table I: Cyclodextrin Complex Moisture Level

| Sample | % Moisture |
|---|---|
| Spray Dry Process Sample A | 4.4 |
| Spray Dry Process Sample B | 3.7-4.5 |
| Spray Dry Process Sample C | 5.3 |
| Extruder Process Sample A | 27.87 |
| Extruder Process Sample B | 27.97 |
| Extruder Process Sample C | 24.00 |

Water content, USP (United States Pharmacopeia, current as of August 1, 2006) <921> Method I is the analytical method for determining cyclodextrin complex moisture level, as shown in Table I.

As one can see from Table 1, the moisture level directly manifested by these two methods is dramatically different. It should be understood that this comparison is not intended to disclaim kneading/extruder processes from appended claims that do not specify a particular complex formation process. Rather, a kneading and extrusion method, or other method forming particles/complexes with higher than desired moisture levels, could utilize additional processing after their initial formation. For example, extruded complexes may be processed through an oven or dryer, or exposed to a controlled environment for a period of time.

Although not wishing to be bound by theory, it is believed that cyclodextrin particles/complexes having a relatively high moisture level have an increased tendency to agglomerate. The agglomerated particles may reach a size so as to become perceptible by a consumer; that is, a consumer may characterize the composition as being "gritty." A "gritty" composition may not be desirable to some consumers. Microbial growth is another potential disadvantage associated with employing cyclodextrin particles/complexes with relatively high moisture levels into a final composition depending on the remaining ingredients of the composition and/or storage parameters.

The efficiency or level of complexing with a perfume composition is another parameter of cyclodextrin complexes that can vary greatly depending on the manufacturing techniques employed. Put another way, the percent of perfume composition that is associated with the interior of a cyclodextrin molecule compared to the percent of perfume composition that is associated with the exterior of the cyclodextrin complex. The perfume composition that is on the exterior region of the complex is essentially free to be expressed without the requirement of a triggering mechanism. The probability that a consumer perceives the perfume composition prior to a triggering mechanism increases as the level of free perfume composition increases. And perception of a perfume composition prior to a triggering mechanism may not be desired depending on the overall composition design and targeted benefit associated with employment of the cyclodextrin complexes. The percent of perfume composition that is complexed with cyclodextrin can be, for example, greater than about 75%, in some instances greater than about 90%, and in other instances greater than about 95%. It should be understood that these levels of perfume complexation are directly associated with the complex formation process itself; the percentages do not represent a formulation design of adding a first percentage of perfume composition via a cyclodextrin complex and adding a second percentage of neat perfume composition.

Spray drying a slurry or mixture of cyclodextrin-perfume complexes is one manufacturing technique capable of producing cyclodextrin complexes having the above-noted levels of perfume composition complexation. Table II below provides a comparison of spray dried cyclodextrin complexes versus complexes formed via an extruder process (kneading).

Table II: Percent of Perfume Composition Loading in Cyclodextrin Complexes

| Sample | Complexation Efficiency |
| --- | --- |
| Spray Dry Process Sample A | 96.6 |
| Spray Dry Process Sample B | 96.8 |
| Spray Dry Process Sample C | 96.2 |
| Extruder Process Sample A | 60.77 |
| Extruder Process Sample B | 65.47 |
| Extruder Process Sample C | 67.07 |

One can see from Table II that spray drying is capable of producing cyclodextrin complexes with very little free perfume composition as compared to a kneading/extruder process. The skilled artisan should appreciate that the comparison provided in Table II is not intended to disclaim kneading/extruder processes from appended claims that do not specify a particular complex formation process. Rather, additional processing steps may, for example, be employed to eliminate free perfume composition associated with extruded complexes prior to their inclusion into a composition.

The analytical method for determining the percent of perfume composition complexed, as shown in Table II, determines the free perfume composition level in the complex by dissolving a sample in tetrahydrofuran (THF) adding an internal standard, and analyzing by capillary gas chromatography (GC). The complexed perfume composition level is measured by extracting the same sample in acetone containing an internal standard, and analyzing by GC.

$$\text{Complexation Efficiency} = \% \text{ Complexed} / [\% \text{ Complexed} + \% \text{ Free}]$$

The cyclodextrin complexes may be coated to minimize premature release/activation. Generally, any material that is capable of resisting water penetration is suitable. The coating material may include, for example, hydrocarbons, waxes, petrolatum, silicones, silicone derivatives, partially or fully esterified sucrose esters, and polyglycerol esters. Using petrolatum as an example, a coating process may include combining cyclodextrin complexes with petrolatum at a ratio of about 1:1, for example, and then mixing until the complexes are satisfactorily coated.

Perfume Compositions for Perfume-Cvclodextrin Complexes

perfume composition for perfume-cyclodextrin complexes comprises perfume raw materials. At least a portion of the perfume raw materials for perfume-cyclodextrin complexes may have a cyclodextrin binding coefficient of about 3.0 or less; about 2.5 or less, about 2.0 or less, about 1.0 or less, to about -2. Some of the perfume raw material may have a cLogP of about 2.5 or less, about 2.0 or less, about 1.5 or less, about 1.0 or less, to about -3. Some of the perfume raw materials may have a weight average molecular weight of about 200 Daltons or less, about 180 Daltons or less, about 150 Daltons or less, about 100 Daltons or less, to about 50 Daltons. A perfume raw material will have an odor detection threshold. At least a portion of the perfume raw materials for perfume-cyclodextrin complexes will have an odor detection threshold of about 7 - log molar concentration or greater; about 8 -log molar concentration or greater; about 9 -log molar concentration or greater; to about 11.5 -log molar concentration.

The perfume composition for perfume-cyclodextrin complexes comprises about 10% or more, by weight of the perfume, of perfume raw materials which have a cyclodextrin binding coefficient of about 3.0 or less, a cLogP of about 2.5 or less, and a weight average molecular weight of about 200 Daltons or less. Going further, the perfume composition for perfume-cyclodextrin complexes comprise 50% or more, up to 100%; of perfume raw materials which have a cyclodextrin binding coefficient of about 3.0 or less, a cLogP of about 2.5 or less, and a weight average molecular weight of about 200 Daltons or less. In addition, a perfume composition for perfume-cyclodextrin complexes may also include perfume raw materials with an odor detection threshold of about 7 -log molar concentration.

A representative, non-limiting, list of perfume raw materials that have a cyclodextrin binding coefficient of about 3.0 or less, a cLogP of about 2.5 or less, and a weight average molecular weight of about 200 Daltons or less is included in the chart below.

| CAS Number | Name | LogP (v3.0 ) | Formula Weight | Odor Detection Threshold , Neural Net model | bCD Complex Stability Constant |
|---|---|---|---|---|---|
| 10031-96-6 | eugenyl formate | 2.35 | 192.214 | 8.83926 | 2.71447114 2 |
| 100-52-7 | Benzaldehyde | 1.4 | 106.124 | 7.44864 | 2.18834196 2 |
| 10094-40-3 | 2-hexen-1-yl acetate | 2.21 | 142.197 | 8.20415 | 1.45329211 2 |
| 101-39-3 | alpha-methyl cinnamaldehyde | 2.18 | 146.188 | 8.83421 | 1.07782473 2 |
| 101-41-7 | Methyl phenylacetate | 1.89 | 150.177 | 8.02162 | 2.14282262 |
| 101-48-4 PADMA | Viridine | 1.65 | 166.219 | 8.01031 | 2.26010286 5 |
| 101-97-3 | Ethyl 2-phenylacetate | 2.39 | 164.204 | 8.63019 | 2.24876912 3 |
| 103-25-3 | methyl hydrocinnamate | 2.04 | 164.204 | 8.20423 | 2.24137482 8 |
| 103-26-4 | Methyl cinnamate | 2.44 | 162.188 | 8.96566 | 2.07152717 9 |
| 103-45-7 phenyl ethyl acetate | 2-Phenylethyl acetate | 2.07 | 164.204 | 8.15422 | 1.54434098 6 |
| 103-54-8 | Cinnamyl acetate | 2.49 | 176.215 | 8.51095 | 1.52537684 6 |
| 104-09-6 | lilac acetaldehyde | 2.12 | 134.177 | 9.36476 | 2.67052556 9 |
| 104-20-1 frambinone | 4-(p-Methoxyphenyl)-2-butanone | 1.88 | 178.23 | 8.85505 | 1.71919798 8 |
| 104-46-1 | Anethole | 2.43 | 148.204 | 8.79496 | 2.33828488 4 |
| 104-50-7 | gamma-Octalactone | 2.06 | 142.197 | 8.29615 | 2.93868131 8 |
| 104-53-0 | 3-phenyl propionaldehyde | 1.65 | 134.177 | 8.94528 | 2.46698172 2 |
| 104-54-1 | Cinnamic alcohol | 1.68 | 134.177 | 8.58181 | 2.15244176 2 |
| 104-55-2 | Cinnamic aldehyde | 1.92 | 132.162 | 8.56091 | 2.36515598 7 |
| 104-62-1 | Phenethyl formate | 1.82 | 150.177 | 8.1025 | 2.32058558 9 |
| 104-64-3 | 3-phenyl propyl formate | 2.22 | 164.204 | 8.51464 | 2.45601200 6 |
| 105-01-1 | Isobutyl furylpropionate | 2.34 | 196.246 | 8.5982 | 2.30094011 1 |
| 10521-96-7 | Styryl acetate | 2.3 | 162.188 | 8.59975 | 1.46959652 4 |
| 105-86-2 | geranyl formate | 2.44 | 182.262 | 8.48662 | 1.85390519 2 |
| 10606-47-0 | 3-Hepten-1-ol | 1.79 | 114.187 | 8.47454 | 2.11095399 5 |
| 106-22-9 | Citronellol | 2.49 | 156.267 | 8.3712 | 0.64479173 3 |
| 106-24-1 | trans-Geraniol | 1.95 | 154.252 | 9.35662 | 2.13328252 3 |
| 106-25-2 | Nerol | 1.95 | 154.252 | 9.35662 | 2.13328252 3 |
| 106-26-3 | Neral | 2.33 | 152.236 | 8.48283 | -1.81879189 |
| 106-72-9 melonal | melon heptenal | 2.09 | 140.225 | 8.08951 | 0.64121203 4 |
| 107-03-9 | Propyl mercaptan | 1.87 | 76.1562 | 9.03723 | 0.65049748 2 |
| 1073-26-3 | 2-Propionylpyrrole | 1.37 | 123.154 | 8.12678 | 1.88368143 1 |
| 110458-85-0 | 5,6-Dimethyl-1-(1-methylethenyl) bicyclo[2.2.1]hept -5-ene-2-methanol | 2.36 | 192.3 | 9.46428 | 1.27164025 5 |

(continued)

| CAS Number | Name | LogP (v3.0 ) | Formula Weight | Odor Detection Threshold , Neural Net model | bCD Complex Stability Constant |
|---|---|---|---|---|---|
| 1123-85-9 | Hydratopic alcohol | 1.85 | 136.193 | 8.18715 | 1.9930676 |
| 1131-62-0 | 3,4-Dimethoxyacetophenone | 1.7 | 180.203 | 8.15118 | 1.63187244 |
| 116-26-7 | Safranal | 2.4 | 150.22 | 8.54472 | 1.29821750 1 |
| 118-93-4 | 2-Hydroxyacetophenone | 1.97 | 136.15 | 8.14874 | 1.38361244 9 |
| 1197-06-4 | cis-carveol | 1.86 | 152.236 | 8.60293 | 0.3234278 |
| 1205-17-0 Helional | ocean propanal | 1.77 | 192.214 | 8.89285 | 2.67119572 5 |
| 120-58-1 | Isosafrol | 2.01 | 162.188 | 8.45077 | 2.52438973 2 |
| 120-72-9 | Indole | 2.34 | 117.15 | 8.19942 | 2.18904082 6 |
| 120-75-2 | 2-Methylbenzothiazole | 2.14 | 149.21 | 8.12212 | 2.82506661 8 |
| 121-32-4 | Ethyl vanillin | 1.53 | 166.176 | 10.3178 | 2.40833498 4 |
| 121-33-5 | Vanillin | 1.04 | 152.149 | 9.92835 | 2.36289444 2 |
| 121-98-2 | Methyl p-anisate | 1.99 | 166.176 | 8.53827 | 2.05017347 5 |
| 122-63-4 | Benzyl propionate | 2.24 | 164.204 | 8.28879 | 2.00750019 |
| 122-72-5 | 3-phenyl propyl acetate | 2.48 | 178.23 | 8.70414 | 1.72908199 1 |
| 122-78-1 | phenyl acetaldehyde | 1.46 | 120.151 | 8.39657 | 2.29743529 4 |
| 123-08-0 | p-Hydroxybenzaldehyde | 1.29 | 122.123 | 9.3415 | 2.28239067 |
| 123-11-5 | para-anisaldehyde | 1.53 | 136.15 | 7.71998 | 2.29432713 8 |
| 123-92-2 | Isoamyl acetate | 1.87 | 130.186 | 7.11654 | 1.32928648 5 |
| 13327-56-5 | Ethyl 3-methylthiopropionate | 1.47 | 148.22 | 8.08772 | 1.87832269 7 |
| 134-20-3 | Methyl anthranilate | 1.58 | 151.165 | 8.21638 | 1.68962441 1 |
| 13494-08-1 | 1,2-Cyclopentanedione, 3-ethyl- | 0.5 | 126.155 | 8.28744 | 2.71644396 1 |
| 134-96-3 | Syringaldehyde | 0.94 | 182.176 | 9.8947 | 2.47833318 2 |
| 13678-68-7 | furfuryl thioacetate | 1.09 | 156.199 | 8.10673 | 1.33394981 8 |
| 13679-85-1 | blackberry thiophenone | 0.73 | 116.178 | 8.43566 | 2.06310637 1 |
| 140-39-6 | p-Cresyl acetate | 2.17 | 150.177 | 8.09518 | 1.66759911 4 |
| 14049-11-7 | linalool oxide (pyranoid) | 1.89 | 170.251 | 8.451 | 2.62000731 4 |
| 141-27-5 | Geranial | 2.33 | 152.236 | 8.48283 | -1.81879189 |
| 142653-61-0 | Parmanyl | 1.75 | 153.224 | 8.13267 | 2.04540237 3 |
| 142-83-6 | Sorbinaldehyde | 1.29 | 96.1286 | 8.57095 | 2.28582033 2 |
| 14360-50-0 | Pentyl 2-furyl ketone | 2.49 | 166.219 | 9.38547 | 2.44441075 6 |
| 150-19-6 | m-Guaiacol | 1.39 | 124.139 | 8.16161 | 2.02364275 1 |
| 1504-55-8 cypriol | alpha-Methylcinnamic alcohol | 1.73 | 148.204 | 8.68023 | 0.74211388 |
| 15111-56-5 | Ethyl cyclohex-3-enecarboxylate | 1.86 | 154.208 | 8.46966 | 2.78144272 7 |
| 1516-17-2 | 2,4-Hexadienyl acetate | 1.75 | 110.155 | 8.29887 | 1.35905361 6 |

(continued)

| CAS Number | Name | LogP (v3.0 ) | Formula Weight | Odor Detection Threshold , Neural Net model | bCD Complex Stability Constant |
|---|---|---|---|---|---|
| 15174-69-3 | 4-Hydroxy-3-methyl benzaldehyde | 1.63 | 136.15 | 10.2521 | 2.24488128 9 |
| 15186-51-3 | Furan, 3-methyl-2-(3-methyl-2-butenyl)- | 2.04 | 150.22 | 8.25595 | 0.46159406 6 |
| 1540-28-9 | n-Pentyl acetoacetate | 1.63 | 172.224 | 8.03665 | 1.78781329 3 |
| 1552-67-6 | Ethyl 2-hexenoate | 2.49 | 142.197 | 8.30088 | 2.11894523 5 |
| 15679-12-6 | 2-Ethyl-4-methylthiazole | 1.69 | 127.204 | 8.31411 | 2.13003539 5 |
| 15679-13-7 | tropical thiazole | 2.12 | 141.231 | 8.2543 | 2.33026124 3 |
| 16251-77-7 | Trifernal | 2.28 | 148.204 | 8.87214 | 2.50625532 |
| 1646-26-0 | Coumarone | 1.9 | 160.172 | 8.63836 | 1.90139916 4 |
| 16491-25-1 | 2,4-Hexadienyl propionate | 2.44 | 154.208 | 8.71795 | 1.97128226 2 |
| 1679-07-8 | Cyclopentyl mercaptan | 2.24 | 102.194 | 9.089 | 1.46723866 3 |
| 1679-09-0 | 2-Methyl-2-butanethiol | 2.45 | 104.21 | 9.16396 | 0.79041982 |
| 16957-70-3 Strawberrif f | trans-2-Methyl-2-pentenoic acid | 1.33 | 114.144 | 8.77632 | 0.64676689 9 |
| 1708-34-5 | 2-Hexyl-1,3-dioxolane | 2.17 | 158.24 | 8.11029 | 2.55533387 |
| 1708-81-2 | cis-3-Hepten-1-ol | 1.79 | 114.187 | 8.47454 | 2.11095399 5 |
| 1708-82-3 | 3-Hexenyl acetate | 2.18 | 142.197 | 8.16238 | 1.47803713 2 |
| 17102-64-6 | Trans,trans-2,4-Hexadien-1-01 | 0.96 | 98.1444 | 8.22126 | 2.05790582 1 |
| 1754-62-7 | METHYL TRANS-CINNAMATE, 99% | 2.44 | 162.188 | 8.96566 | 2.07152717 9 |
| 1759-28-0 | 4-Methyl-5-vinylthiazole | 1.51 | 125.188 | 8.5586 | 1.6235833 |
| 17626-75-4 | 2-Propylthiazole | 1.51 | 127.204 | 8.23479 | 1.79492639 8 |
| 18031-40-8 | (S),(-)-Perillaaldehyde | 2.34 | 150.22 | 9.79611 | 1.84742265 6 |
| 18277-27-5 | 2-(1-Methylpropyl)thiazole | 1.9 | 141.231 | 8.25112 | 1.70589046 |
| 18479-68-0 | (+)-P-MENTH-1-EN-9-OL, 97%, MIXTURE OF ISOMERS | 2.26 | 154.252 | 8.86753 | 1.65686528 5 |
| 18640-74-9 | Isobutyl thiazole | 1.92 | 141.231 | 8.28889 | 2.02435600 6 |
| 18829-55-5 | trans-2-Heptenal | 2.1 | 112.171 | 8.76119 | 2.32645722 1 |
| 18881-04-4 | (1S)-(-)-cis-Verbenol | 2.03 | 152.236 | 8.08642 | 2.61062368 2 |
| 189440-77-5 | Anapear | 2.3 | 154.208 | 8.78281 | 2.20249931 2 |
| 1901-38-8 | alpha-Campholenic alcohol | 2.03 | 154.252 | 8.08006 | 1.31977542 6 |
| 19788-49-9 | Ethyl 2-mercaptopropionate | 1.41 | 134.193 | 8.39452 | 0.99280932 6 |
| 19819-98-8 | 2-Methylphenethyl alcohol | 1.66 | 136.193 | 8.45733 | 2.35703375 3 |
| 2046-17-5 | Methyl 4-phenylbutyrate | 2.46 | 178.23 | 8.75021 | 2.37117683 8 |
| 20474-93-5 | Allyl crotonate | 1.63 | 126.155 | 8.28675 | 2.23707170 8 |
| 2051-78-7 | Allyl butyrate | 1.88 | 128.171 | 8.16812 | 2.20975650 5 |
| 2051-96-9 | Benzyl lactate | 1.35 | 180.203 | 8.14769 | 1.69609981 |

(continued)

| CAS Number | Name | LogP (v3.0 ) | Formula Weight | Odor Detection Threshold , Neural Net model | bCD Complex Stability Constant |
|---|---|---|---|---|---|
| 20665-85-4 | Vanillin isobutyrate | 1.92 | 222.24 | 8.19703 | 2.20204286 7 |
| 2111-75-3 | perillaldehyde | 2.34 | 150.22 | 9.79611 | 1.84742265 6 |
| 2142-94-1 | Neryl Formate | 2.44 | 182.262 | 8.48662 | 1.85390519 2 |
| 2179-58-0 | Allyl methyl disulfide | 1.9 | 120.227 | 8.59268 | 1.43625368 2 |
| 2179-60-4 | Methyl propyl disulfide | 2.28 | 122.243 | 8.56359 | 1.97210861 8 |
| 21835-00-7 | 2-Cyclopenten-1-one, 2-hydroxy-3,4-dimethyl- | -0.02 | 126.155 | 8.9115 | 0.75713068 |
| 21835-01-8 | 3-Ethyl-2 -hyd roxy-2 -cyclopenten-1-one | 0.06 | 126.155 | 8.78501 | 2.40810278 1 |
| 22104-78-5 | 2-Octenol-1 | 2.27 | 128.214 | 8.80636 | 2.23545411 9 |
| 2217-33-6 | Tetrahydrofurfuryl butyrate | 1.54 | 172.224 | 8.39609 | 2.21972031 9 |
| 22451-63-4 | Allo-ocimenol | 2.42 | 152.236 | 8.5083 | 0.98832007 4 |
| 22460-95-3 | 7-Octene-1,6-diol, 3,7-dimethyl- | 1.33 | 172.267 | 8.27199 | 0.79362903 6 |
| 22924-15-8 | 3-Ethoxybenzaldehyde | 1.99 | 150.177 | 8.14443 | 2.32721253 6 |
| 22927-13-5 | 2-Ethylbenzaldehyde | 2.06 | 134.177 | 8.77955 | 2.52759276 8 |
| 2305-21-7 | 2-hexen-1-ol | 1.3 | 100.16 | 8.08924 | 2.05671951 7 |
| 23495-12-7 | Phenoxyethyl propionate | 2.43 | 194.23 | 8.91769 | 1.78472652 9 |
| 23911-56-0 | Nerolione | 2.02 | 174.199 | 8.74265 | 2.04071556 4 |
| 2445-83-2 | 7-Methylcoumarin | 2.42 | 160.172 | 8.7864 | 2.77624082 |
| 2463-63-0 | Butylacrolein | 2.1 | 112.171 | 8.76119 | 2.32645722 1 |
| 2497-18-9 | 2-Hexen-1-yl acetate | 2.21 | 142.197 | 8.20415 | 1.45329211 2 |
| 2555-49-9 | Ethyl phenoxyacetate | 2.04 | 180.203 | 8.36377 | 1.92718106 |
| 26553-46-8 | Ethyl trans-3-hexenoate | 2.25 | 142.197 | 8.34357 | 2.14403463 9 |
| 2719-08-6 | N-Acetyl methyl anthranilate | 1.21 | 193.202 | 8.00148 | 1.47732197 6 |
| 27829-72-7 | Ethyl trans-2-hexenoate | 2.49 | 142.197 | 8.30088 | 2.11894523 5 |
| 27939-60-2 triplal extra | Vertoliff | 1.8 | 138.209 | 9.23879 | 1.71162312 3 |
| 28069-72-9 | (2E,6Z)-Nona-2,6-dien-1-ol | 2.43 | 140.225 | 9.5865 | 2.24137362 2 |
| 28977-58-4 | Ocimenol | 2.02 | 152.236 | 8.7107 | -0.59222945 |
| 29414-56-0 | 2,6-Dimethyl-1,5,7-octatrienol-3 | 1.96 | 152.236 | 8.88676 | 0.75677855 8 |
| 29548-14-9 | p-Menth-1-ene-9-al | 2.24 | 152.236 | 9.40193 | 1.85419777 9 |
| 30361-28-5 | 2,4-Octadien-1-al | 2.45 | 124.182 | 9.33257 | 2.32496104 6 |
| 30954-98-4 | Propyl anthranilate | 2.47 | 179.218 | 8.87684 | 1.86814504 4 |
| 3194-17-0 | 2-Pentanoylfuran | 1.99 | 152.193 | 8.9735 | 2.39891684 8 |
| 32272-48-3 | 4-Ethyl-2-methylthiazole | 1.7 | 127.204 | 8.31539 | 2.24879066 9 |
| 32764-98-0 | Jasmolactone | 2.36 | 168.235 | 8.717 | 2.95677912 1 |
| 33467-73-1 | cis-3-Hexenyl formate | 1.69 | 128.171 | 8.21587 | 2.24507821 5 |

(continued)

| CAS Number | Name | LogP (v3.0 ) | Formula Weight | Odor Detection Threshold , Neural Net model | bCD Complex Stability Constant |
|---|---|---|---|---|---|
| 3391-86-4 | 1-Octenol-3 | 2.36 | 128.214 | 8.28727 | 2.19425030 2 |
| 3581-91-7 | 4,5-Dimethylthiazole | 0.91 | 113.177 | 8.0998 | 1.30378168 8 |
| 3583-00-4 | 4,4-Dimethyl-5-isopropyl-1,3-dioxolane | 1.92 | 158.24 | 8.99459 | 1.97597132 7 |
| 35926-04-6 | 1-Hexen-3-yl acetate | 2.31 | 142.197 | 8.02087 | 1.68283261 |
| 36701-01-6 | Furfuryl valerate | 1.89 | 182.219 | 8.38621 | 2.12370904 7 |
| 36806-46-9 | 2,6-Dimethyl-6-hepten-1-ol | 2.4 | 142.241 | 8.07086 | 0.75889681 1 |
| 3681-71-8 | cis-3-Hexenyl acetate | 2.18 | 142.197 | 8.16238 | 1.47803713 2 |
| 3681-82-1 | trans-3-Hexenyl acetate | 2.18 | 142.197 | 8.16238 | 1.47803945 2 |
| 36880-33-8 | 5-Ethyl-2-thiophenecarbaldehyde | 1.85 | 140.2 | 8.18526 | 2.64423037 6 |
| 37973-51-6 | 2-Phenyl-1(2)propenyl-1 ester | 2.47 | 176.215 | 8.82371 | 0.44183192 4 |
| 38142-45-9 | 3-Cyclohexene-1-ethanol, 4-methyl-.beta.-methylene-, (R)- | 1.84 | 152.236 | 8.61826 | 1.58143019 6 |
| 39252-02-3 | Furfuryl hexanoate | 2.38 | 196.246 | 8.80269 | 2.17419392 1 |
| 39677-52-6 | 3-METHOXY CINNAMALDEHYDE | 1.86 | 162.188 | 8.83589 | 2.48765937 8 |
| 40010-99-9 | 3-Acetyl-5-butyldihydro-2(3H)-furanone | 1.71 | 184.235 | 8.57113 | 2.57881260 4 |
| 40790-29-2 | Pyrazine, 3-butyl-2,5-dimethyl- | 2.29 | 164.25 | 8.18163 | 2.48191224 |
| 409-02-9 | Methyl Heptenone | 2.27 | 126.198 | 8.5811 | 2.38462734 3 |
| 4175-66-0 | 2,5-Dimethylthiazole | 0.94 | 113.177 | 8.08045 | 1.63315635 7 |
| 4180-23-8 | (E)-anethol | 2.43 | 148.204 | 8.79496 | 2.33828488 4 |
| 41847-88-5 | Phenylethyl oxy-acetaldehyde | 1.55 | 164.204 | 8.60614 | 2.34043786 3 |
| 42348-12-9 | 3-Ethyl-2-hydroxy-4-methylcyclopent-2-en-1-one | 0.54 | 140.182 | 9.09988 | 2.57794662 2 |
| 4313-03-5 | (E,E)-2,4-heptadien-1-al | 1.98 | 110.155 | 8.99985 | 2.28539782 4 |
| 4364-06-1 | Cinnamic aldehyde dimethyl acetal | 2.02 | 178.23 | 8.43761 | 2.03344013 6 |
| 4501-58-0 | Campholene aldehyde | 2.2 | 152.236 | 8.30764 | 1.42588904 6 |
| 4634-89-3 | cis-4-Hexenal | 1.05 | 98.1444 | 9.24362 | 2.25752596 3 |
| 4643-25-8 | 2-Hepten-4-one | 1.85 | 112.171 | 8.31026 | 2.21288655 9 |
| 4643-27-0 | 2-Octen-4-one | 2.42 | 126.198 | 8.70341 | 2.43020564 3 |
| 473-67-6 | Verbenol | 2.03 | 152.236 | 8.08642 | 2.61062368 2 |
| 4748-78-1 | 4-Ethylbenzaldehyde | 2.39 | 134.177 | 9.18923 | 2.53840536 6 |
| 491-04-3 | Piperitol | 2.4 | 154.252 | 8.70061 | 1.72126892 8 |
| 491-09-8 | piperitenone | 2.33 | 150.22 | 8.39506 | 1.19626340 4 |
| 491-31-6 | Isocoumarin | 1.69 | 146.145 | 8.63271 | 2.45114999 8 |

(continued)

| CAS Number | Name | LogP (v3.0 ) | Formula Weight | Odor Detection Threshold , Neural Net model | bCD Complex Stability Constant |
|---|---|---|---|---|---|
| 491-35-0 | Lepidine | 2.46 | 143.188 | 8.13439 | 2.43836142 9 |
| 4940-11-8 | ethyl maltol | 0.17 | 140.138 | 7.43727 | 1.94452587 3 |
| 496-77-5 | Butyroin | 1.29 | 144.213 | 8.35862 | 2.21548054 3 |
| 499-44-5 | Hinokitiol | 1.35 | 164.204 | 9.32432 | 2.70664289 7 |
| 50888-63-6 | Pyrazine, 2-butyl-3,5-dimethyl- | 2.3 | 164.25 | 8.18507 | 2.27450062 3 |
| 53046-97-2 | cis-3, cis-6-nonadienol | 2.45 | 140.225 | 9.51927 | 2.16222354 8 |
| 53398-78-0 | trans-2-Hexenyl formate | 1.71 | 128.171 | 8.31245 | 2.23325164 6 |
| 53399-81-8 | Ethyl 2-methyl-4-pentenoate | 2.26 | 142.197 | 8.16105 | 2.07534686 9 |
| 536-50-5 | 1-(4-Methylphenyl)ethanol | 2 | 136.193 | 8.06804 | 2.38996557 4 |
| 536-59-4 | Perillyl alcohol | 1.83 | 152.236 | 8.5842 | 1.69222565 |
| 536-60-7 | Cumic alcohol | 2.39 | 150.22 | 8.68229 | 2.38635265 3 |
| 5392-40-5 | citral | 2.33 | 152.236 | 8.48283 | -1.81879189 |
| 5396-89-4 | Benzyl acetoacetate | 1.43 | 192.214 | 8.04859 | 1.4535056 |
| 5406-12-2 | p-Methylhydrocinnamic aldehyde | 2.19 | 148.204 | 9.5736 | 2.83971467 |
| 541-58-2 | 2,4-Dimethylthiazole | 1.24 | 113.177 | 8.08092 | 1.89355759 3 |
| 5426-78-8 | Acetaldehyde phenyl ethyl acetal | 2.22 | 166.219 | 8.55675 | 1.82846441 7 |
| 5462-06-6 | Canthoxal | 2.16 | 178.23 | 8.80131 | 2.49489353 3 |
| 5466-06-8 | Ethyl 3-mercaptopropionate | 1.36 | 134.193 | 8.91613 | 1.24698749 3 |
| 5471-51-2 | Raspberry ketone | 1.58 | 164.204 | 7.67158 | 1.69633171 5 |
| 554-14-3 | 2-Methylthiophene | 2.06 | 98.1624 | 8.11204 | 1.51680517 6 |
| 55722-59-3 | 3,6-Octadienal, 3,7-dimethyl- | 2.34 | 152.236 | 8.50763 | 1.88980115 3 |
| 5577-44-6 | 2,4-Octadienal | 2.45 | 124.182 | 9.33257 | 2.32496104 6 |
| 5660-60-6 | Cinnamaldehyde ethylene glycol acetal | 2.15 | 176.215 | 8.04159 | 2.15815099 |
| 56805-23-3 | trans-3, cis-6-nonadienol | 2.45 | 140.225 | 9.51927 | 2.16222354 8 |
| 57266-86-1 | 2-Heptenal, (2Z)- | 2.1 | 112.171 | 8.76119 | 2.32645722 1 |
| 57500-00-2 | Methyl furfuryl disulfide | 1.92 | 160.249 | 8.18632 | 2.38204616 7 |
| 579-74-8 | o-Acetylanisole | 1.55 | 150.177 | 8.39887 | 1.55788362 9 |
| 58461-27-1 | Lavandulol | 1.95 | 154.252 | 8.97983 | -1.81754606 |
| 585-74-0 | 3-Methylacetophenone | 2.27 | 134.177 | 8.22927 | 1.65423043 2 |
| 589-18-4 | p-Tolyl alcohol | 1.62 | 122.166 | 8.01143 | 2.34757410 4 |
| 59020-85-8 | Furfuryl thiopropionate | 1.61 | 170.226 | 8.45315 | 2.15610657 4 |
| 59021-02-2 | 2-Mercaptomethylpyrazine | 0.34 | 126.176 | 8.25962 | 0.66487833 2 |
| 5910-85-0 | 2,4-Heptadienal | 1.98 | 110.155 | 8.99985 | 2.28539782 4 |
| 5912-86-7 | cis-iso-Eugenol | 1.85 | 164.204 | 8.59893 | 2.37510816 4 |
| 5925-68-8 | S-Ethyl benzothioate | 2.21 | 152.211 | 8.7381 | 1.82694472 2 |
| 5932-68-3 | trans-Isoeugenol | 1.85 | 164.204 | 8.59893 | 2.37510816 4 |

(continued)

| CAS Number | Name | LogP (v3.0 ) | Formula Weight | Odor Detection Threshold , Neural Net model | bCD Complex Stability Constant |
|---|---|---|---|---|---|
| 606-27-9 | Methyl 2-nitrobenzoate | 1.57 | 181.148 | 8.45477 | 2.25159889 9 |
| 606-45-1 | Methyl o-methoxybenzoate | 1.79 | 166.176 | 8.55546 | 2.14578607 7 |
| 613-70-7 | Guaiacyl acetate | 1.55 | 166.176 | 8.18455 | 1.56646107 6 |
| 616-44-4 | 3-Methylthiophene | 2.23 | 98.1624 | 8.50809 | 1.51680517 6 |
| 6191-71-5 | cis-4-Hepten-1-ol | 1.77 | 114.187 | 8.46019 | 2.11095399 5 |
| 6192-44-5 | beta-Phenoxy ethyl acetate | 1.87 | 180.203 | 8.50733 | 1.25661617 1 |
| 61931-81-5 | cis-3-Hexenyl lactate | 1.34 | 172.224 | 8.19554 | 1.75618591 4 |
| 620-23-5 | meta-tolyl aldehyde | 2.13 | 120.151 | 8.79305 | 2.38212641 4 |
| 623-15-4 | 4-(2-Furyl)-3-buten-2-one | 1.7 | 136.15 | 8.41807 | 1.37810431 |
| 624-92-0 | Dimethyl disulfide | 1.06 | 94.1894 | 8.63915 | 0.26857377 7 |
| 6290-14-8 | Cyclopentyl isobutyrate | 2.29 | 156.224 | 8.41781 | 2.08155627 6 |
| 6314-97-2 | Phenylacetaldehyde diethyl acetal | 2.29 | 194.273 | 9.02295 | 2.37014962 6 |
| 637-65-0 | tetrahydrofurfuryl propionate | 0.93 | 158.197 | 8.0157 | 2.06615661 9 |
| 638-02-8 | 2,5-Dimethylthiophene | 2.36 | 112.189 | 8.63601 | 2.04405336 2 |
| 64988-06-3 | Ethyl 2-methoxybenzyl ether | 1.98 | 166.219 | 8.22901 | 2.27345362 7 |
| 65405-67-6 | p-M ethoxy-a lpha-methyl cinnamaldehyde | 2 | 176.215 | 8.85096 | 1.16169947 5 |
| 65405-73-4 | Geranyl oxyacetaldehyde | 2.32 | 196.289 | 8.71128 | 1.87724548 9 |
| 67028-40-4 | Ethyl (p-tolyloxy)acetate | 2.49 | 194.23 | 8.45474 | 2.18439262 1 |
| 6728-26-3 | Trans-2-Hexenal | 1.57 | 98.1444 | 8.41218 | 2.25752596 3 |
| 6728-31-0 | cis-4-Heptenal | 1.85 | 112.171 | 9.51008 | 2.32645722 1 |
| 67633-97-0 | 3-Mercapto-2-pentanone | 1.37 | 118.193 | 8.85981 | 0.23467913 6 |
| 67634-07-5 | 3,5,6-Trimethyl-3-cyclohexene-1-carbaldehyde | 2.37 | 152.236 | 8.63004 | 1.97142579 4 |
| 67634-16-6 | Floralol | 1.83 | 140.225 | 8.37888 | 1.49812766 8 |
| 67634-17-7 | 2,4-Dimethyl-3-cyclohexene-1-methanol | 1.81 | 140.225 | 8.50806 | 1.61306393 6 |
| 67746-30-9 | trans-2-Hexenal diethyl acetal | 2.34 | 172.267 | 8.19269 | 2.12984062 5 |
| 67801-65-4 | 3,6-ivy carbaldehyde | 1.8 | 138.209 | 9.24685 | 2.08978317 5 |
| 67845-46-9 | p-Methyl phenoxy acetaldehyde | 1.76 | 150.177 | 8.6393 | 2.39631571 1 |
| 6789-80-6 | (Z)-3-hexen-1-al | 1.43 | 98.1444 | 8.974 | 2.25752596 3 |
| 68039-48-5 | Dimethyl cyclohexene carboxaldehyde | 1.82 | 138.209 | 9.17777 | 1.64522337 1 |
| 68039-49-6 Ligustral | 2,4-Dimethyl-3-Cyclohexene-1-carboxaldehyde | 1.78 | 138.209 | 9.23851 | 1.75627497 6 |
| 68133-76-6 | cis-3-Hexenyl pyruvate | 1.9 | 170.208 | 8.49764 | 1.30256675 2 |
| 68737-61-1 | 3,5-ivy carbaldehyde | 1.82 | 138.209 | 9.17777 | 1.64522337 1 |

(continued)

| CAS Number | Name | LogP (v3.0 ) | Formula Weight | Odor Detection Threshold , Neural Net model | bCD Complex Stability Constant |
|---|---|---|---|---|---|
| 698-76-0 | delta-Octalactone | 2.03 | 142.197 | 8.24031 | 2.82791363 1 |
| 699-10-5 | Methyl benzyl disulfide | 2.47 | 170.287 | 8.44764 | 2.95598873 4 |
| 701-70-2 | 1-Phenylbutan-2-ol | 2.21 | 150.22 | 8.58892 | 2.26016176 |
| 7452-79-1 | Ethyl 2-methylbutyrate | 1.91 | 130.186 | 7.26763 | 1.74544754 3 |
| 74-93-1 | Methyl mercaptan | 0.58 | 48.1026 | 8.62509 | 0.43079748 2 |
| 7493-63-2 | Allyl anthranilate | 2.31 | 177.202 | 8.47973 | 1.95348082 4 |
| 7493-71-2 | Allyl tiglate | 1.86 | 140.182 | 8.11576 | 0.69164804 3 |
| 75-08-1 | Ethanethiol | 1.37 | 62.1294 | 8.86633 | 0.63044748 2 |
| 75-18-3 | dimethyl sulfide | 1.24 | 62.1294 | 8.33461 | 0.86448622 3 |
| 75-33-2 | 2-Propanethiol | 1.65 | 76.1562 | 9.25814 | 0.87446503 6 |
| 7540-51-4 | (-)-Citronellol | 2.49 | 156.267 | 8.3712 | 0.64479173 3 |
| 7549-33-9 | Anisyl propionate | 2.23 | 194.23 | 8.44816 | 2.08220943 |
| 75-66-1 | tert-Butyl mercaptan | 1.65 | 90.183 | 9.12728 | 1.12956982 |
| 764-40-9 | 2,4-Pentadienal | 0.7 | 82.1018 | 8.16153 | 2.36826692 1 |
| 76649-25-7 | 3,6-Nonadien-1-ol | 2.45 | 140.225 | 9.51927 | 2.16222354 8 |
| 774-48-1 | Benzaldehyde diethyl acetal | 2.03 | 180.246 | 8.56812 | 2.34797009 2 |
| 7774-74-5 | 2-Thienyl mercaptan | 1.77 | 116.196 | 8.00171 | 0.81012956 8 |
| 7774-79-0 | 4-(p-Tolyl)-2-butanone | 2.46 | 162.231 | 8.63674 | 2.00666781 8 |
| 7774-96-1 | Isoeugenyl formate | 2.35 | 192.214 | 8.83926 | 2.71447114 2 |
| 7786-44-9 | 2,6-Nonadien-1-ol | 2.43 | 140.225 | 9.5865 | 2.24137362 2 |
| 7786-61-0 | 2-Methoxy-4-vinylphenol | 2.24 | 150.177 | 8.70654 | 2.37381744 6 |
| 7786-67-6 | p-Menth-8-en-3-ol (8Cl) | 2.48 | 154.252 | 8.41999 | 2.28583583 6 |
| 81925-81-7 | filbert heptenone | 2.31 | 126.198 | 8.06064 | 1.92004037 9 |
| 84434-18-4 | Gardamide | 2.16 | 191.272 | 8.07979 | 1.98028000 4 |
| 85-91-6 | Dimethyl anthranilate | 2.19 | 165.191 | 8.128 | 2.07755023 2 |
| 870-23-5 | Allyl mercaptan | 1.42 | 74.1404 | 9.00005 | 0.85014748 2 |
| 87-25-2 | Ethyl anthranilate | 2.05 | 165.191 | 8.57858 | 1.84339461 8 |
| 874-66-8 | cinnamon acrolein | 1.29 | 136.15 | 8.09274 | 0.92078825 |
| 881-68-5 | Vanillin acetate | 0.95 | 194.187 | 8.11315 | 1.94259346 6 |
| 89-79-2 | Isopulegol | 2.48 | 154.252 | 8.41999 | 2.28583583 6 |
| 90-02-8 | Salicylaldehyde | 1.4 | 122.123 | 8.94639 | 2.20728487 9 |
| 90-05-1 | Guaiacol | 1.33 | 124.139 | 8.06111 | 1.97727155 5 |
| 90-87-9 | Hydratropaldehyde dimethyl acetal | 2.12 | 180.246 | 8.59983 | 2.23596730 5 |
| 91-64-5 | Coumarin | 1.68 | 146.145 | 8.54868 | 2.46749883 2 |
| 928-94-9 | (Z)-2-hexen-1-ol | 1.3 | 100.16 | 8.08924 | 2.05671951 7 |
| 928-95-0 | (E)-2-hexen-1-ol | 1.3 | 100.16 | 8.08924 | 2.05671951 7 |

(continued)

| CAS Number | Name | LogP (v3.0 ) | Formula Weight | Odor Detection Threshold , Neural Net model | bCD Complex Stability Constant |
|---|---|---|---|---|---|
| 928-96-1 | cis-3-Hexen-1-ol | 1.3 | 100.16 | 8.06286 | 2.05671951 7 |
| 93-16-3 | Methyl isoeugenol | 2.05 | 178.23 | 8.698 | 2.49359976 9 |
| 93-29-8 | Isoeugenyl acetate | 2.17 | 206.241 | 8.37706 | 1.94397986 |
| 93-53-8 | 2-phenyl propionaldehyde | 2.06 | 134.177 | 8.42521 | 2.20578182 5 |
| 93-54-9 | 1-Phenyl-1-propanol | 1.77 | 136.193 | 8.21426 | 2.02509702 1 |
| 93-58-3 | Methyl benzoate | 1.86 | 136.15 | 8.03063 | 1.99686900 2 |
| 93-89-0 | Ethyl benzoate | 2.25 | 150.177 | 8.60489 | 2.18181708 |
| 93893-89-1 | Citronitrile | 2.34 | 171.241 | 8.56816 | 1.27108473 8 |
| 93-92-5 | Styrallyl acetate | 2.2 | 164.204 | 8.17557 | 1.54280022 6 |
| 94087-83-9 | Butanoic acid, 2-methyl-, 2-hexenyl ester, (E)- | 1.6 | 134.236 | 9.31574 | 1.40708743 |
| 94-86-0 | Vanitrope | 2.42 | 178.23 | 8.52869 | 2.39173058 |
| 95-20-5 | 2-Methylindole | 2.43 | 131.177 | 8.53028 | 2.57960408 3 |
| 97-53-0 | Eugenol | 2.21 | 164.204 | 8.57127 | 2.51047130 1 |

One grouping of perfume raw materials that have a cyclodextrin binding coefficient of about 3.0 or less, a cLogP of about 2.5 or less, and a weight average molecular weight of about 200 Daltons or less includes beta gamma hexanol; cis 3 hexenyl acetate; ethyl-2-methyl butyrate; amyl-acetate (isomer blends); vanillin; anethole; methyl isoeugenol; guiacol; floralol; ethyl vanillin; 2,6-nonadien-1-ol; coumarin; and combinations thereof.

Another group of perfume raw materials that have a cyclodextrin binding coefficient of about 3.0 or less, a ClogP of about 2.5 or less, and a weight average molecular weight of about 200 Daltons or less includes ethyl-2-methyl butyrate; beta gamma hexanol; iso amyl acetate; amyl acetate; cis-3-Hexenyl acetate; gamma-Octalactone; ethyl vanillin; vanillin; benzaldehyde; and combinations thereof.

An additional group of perfume raw materials that have a cyclodextrin binding coefficient of about 3.0 or less, a ClogP of about 2.5 or less, and a weight average molecular weight of about 200 Daltons or less includes dimethyl anthranilate; iso-eugenyl acetate; canthoxal; 3,6-nonadien-1-ol, triplal; and combinations thereof.

Some examples of perfume raw materials with an odor detection threshold of 7 -log ppb or more include can be found in the chart above.

Examples Perfume Compositions for Perfume-Cyclodextrin Complexes

Exemplary perfume compositions in accordance with the invention can include:

| Material | % by weight of perfume composition |
|---|---|
| Cis-3-hexen-1-ol | 5-50% |
| Cis-3-hexenyl acetate | 5-50% |
| Ethyl 2-methylbutyrate | 5-50% |
| Isoamyl acetate | 5-50% |
| Vanillin | 5-50% |

Additional information about the perfume raw materials in the example can be found in the table below:

| CAS Number | Name | cLogP | Weight average molecular weight (Dalton) | Odor Detection threshold (-log molar concentration) | Cyclodextrin stability constant (log K) |
|---|---|---|---|---|---|
| 123-92-2 | Isoamyl acetate | 1.87 | 130 | 7.12 | 0.33 |
| 121-33-5 | Vanillin | 1.04 | 152 | 9.93 | 1.36 |
| 7452-79-1 | Ethyl 2-methylbutyrate | 1.91 | 130 | 7.27 | 0.75 |
| 928-96-1 | Cis-3-hexen-1-ol | 1.3 | 100 | 8.06 | 1.06 |
| 3681-71-8 | Cis-3-hexenyl acetate | 2.18 | 142 | 8.16 | 0.48 |

The perfume composition can be made by blending all of the perfume raw materials together until a homogenous solution is formed.

This exemplary composition can then be formed into a cyclodextrin complex by mixing 10 parts cyclodextrin with 10 (or more) parts water, and 1 part (or less) of the perfume composition. After the mixing, the slurry will be more viscous than at the start of mixing - the change in viscosity is believed to be due to the formation of the cyclodextrin perfume complex. The mixture is then dried (or spray dried) to remove the water and leave the cyclodextrin and perfume complex as a powder.

In Vitro Perfume Release Method

Released Perfume (RP) Sample

About 500 milligrams of a cyclodextrin perfume complex is weighed into a glass scintillation vial. About 1 milliliter of water is added to the vial. The vial is then capped tightly and vortexed for about 30 seconds to create a slurry. The RP sample is then placed into a 37 degrees Celsius oven to incubate for 4 hours. The sample vial is removed from the oven and allowed to cool to room temperature. 10 milliliters of hexane is then added to the vial. The vial is capped tightly and mixed by hand shaking for about 10 seconds and then mixed on high speed with a vortex mixer for about 30 seconds to extract perfume components liberated by the water incubation step. After allowing solids to settle, an aliquot of the sample is transferred to a 2 milliliter autosampler vial for analysis.

Total Perfume (TP) Sample

Another 500 milligrams of the same cyclodextrin perfume complex used to create the RP sample is weighed into a scintillation vial. About 10 milliliters of acetone is added to the vial. This sample is then capped tightly and vortexed for about 30 seconds to disperse the sample. The total sample is then placed into a 70 degrees Celsius oven for 4 hours. The sample is removed from the oven and allowed to cool to room temperature. After allowing solids to settle, an aliquot of the sample is transferred to a 2 milliliter autosampler vial for analysis.

Analysis

The RP and TP samples are analyzed using liquid injection gas chromatography with a mass selective detector. The injection port is heated to 270 degrees Celsius and operated in split mode with a split ratio of about 20:1. The carrier gas is helium and delivered at a constant flowrate of about 1.2 milliliters per minute. The oven temperature is ramped from an initial temperature of 50 degrees Celsius to a final temperature of 250 degrees Celsius at a rate of 10 degrees Celsius per minute. The final temperature is held for 2 minutes. The mass selective detector is operated in scanning mode and perfume components are identified using NIST mass spectral library searching. The chromatogram from the TP sample is used to identify a specific mass to charge ratio for each perfume component and extracted ion peak areas for each perfume component are obtained. The RP chromatogram is correspondingly processed.

Results Calculation

Individual perfume component peak areas per unit of sample weight from the RP sample are divided by the corresponding peak areas per unit of sample weight from the TP sample. The resulting ratio is multiplied by 100 to calculate a release percentage for each individual perfume raw material. The release percentages from all perfume components are averaged to calculate a composite release value for a given complex sample.

Spray Composition

A spray composition may be formulated with a perfume-cyclodextrin complex that comprises little or no water. The spray composition may be substantially free of water, meaning that no water is intentionally added to the spray composition. The spray composition may contain from 0 wt. % to about 10 wt. % water or less than 10 wt. % water. The low-water composition may contain additional ingredients such as uncomplexed, free perfume composition, malodor reduction compositions, solvents, propellants, insect repellants, preservatives, surfactants, buffering agents, or other ingredients. The spray composition may be in form of an aerosol spray.

*Perfume-Cyclodextrin Complexes*

The spray composition comprises perfume-cyclodextrin complexes to impart long-lasting scent to the spray composition. The spray composition may comprise up to 5.0 wt.%, or up to 4.0 wt.%, or up to 3.0 wt. % of perfume-cyclodextrin complexes, by weight of the overall spray composition.

*Solvent*

The spray composition may include a solvent. The spray composition may include from 1 to 70 wt % of a solvent.
The solvent system may comprise any organic solvent or mixtures thereof suitable for use in an aerosol composition. The organic solvent system comprises one or more monohydric alcohols, such as low molecular weight monohydric alcohols, including ethanol, propanol, isopropanol, and butanol.
Some low-molecular weight polyhydric alcohols, such as glycols, may also be included in the organic solvent system. The solvent system may comprise one or more non-alcoholic organic solvents, such as acetone, C5-C9 hydrocarbon solvents, esters and diesters.
The spray composition may include a hydrocarbon solvent. The hydrocarbon solvent may be a synthetic isoparaffinic aliphatic hydrocarbon of vapor pressure less than 0.1 mm Hg at 20° C. having a vapor pressure more than 0.03 mm Hg, or having a vapor pressure less than 0.1 mm Hg and more than 0.06 mm Hg.
The spray composition may include a hydrocarbon solvent that is a paraffinic distillate of distillation point less than about 275° C. A hydrocarbon solvent may be a low vapor pressure (LVP) solvent, as defined by the California State Exemption, which exempts LVP-VOCs from the VOC content of over 96 categories of specifically regulated consumer products. This Regulation defines LVP-VOC as a compound or mixture which contains at least one carbon atom and meets one of the following: (a) has a vapor pressure less than 0.1 mm Hg at 20° C. as determined by California Air Resources Board's (ARB) Method 310, section 3.6.3; or (b) is a compound with more than 12 carbon atoms, or a mixture comprised solely of compounds with more than 12 carbon atoms and the vapor pressure is unknown; or (c) is a compound with a boiling point greater than 216° C. as determined by ARB method 310 section 3.6.2; or (d) is the weight percent of a mixture that boils above 216° C. as determined by ARB Method 310, section 3.6.2. Hydrocarbon solvents are commercially available as Isopar® M to be primarily a mixture of C13-C14 isoparaffins, Isopar® P to be primarily a mixture of C12-C20 isoparaffins and Isopar™ V also to be primarily a mixture of C12-C20 isoparaffins ex., ExxonMobil.
In addition to the above discussed organic solvent, the solvent system of the disclosed composition may optionally include a small amount of water. In one embodiment, the disclosed composition comprises no more than about 10 wt % water. When water is present in the composition, the composition may further optionally include one or more corrosion inhibitors to prevent corrosion of the aerosol container or metallic parts of the aerosol device.

*Free Perfume Composition*

The spray composition may comprise a free perfume composition that is not pre-complexed with cyclodextrin. The spray composition may include from about 0.1 wt. % to about 5 wt. %, alternatively about 0.1 wt.% to about 2 wt.%, of an free perfume composition, by weight of the overall spray composition. The free perfume composition may comprise a mixture of one or more perfume raw materials combined to deliver a particular scent experience. The free perfume composition may deliver instantaneous scent, while the perfume-cyclodextrin complexes provide long-lasting scent.

*Malodor Counteractant*

The freshening composition may include malodor counteractants. This may include, without limitation, reactive aldehydes, reactive ketones, amine functional polymers, metal ions, polyols, oxidizing agents, activated carbon, and combinations therof. Malodor counteractants react with or bind to a malodor to neutralize or block the scent of a malodor.

The freshening composition may comprise polyols. Low molecular weight polyols with relatively high boiling points, as compared to water, such as ethylene glycol, diethylene glycol, triethylene glycol, propylene glycol, dipropylene glycol, and/or glycerine may be utilized as a malodor counteractant for improving odor neutralization of the freshening composition. Some polyols, e.g., dipropylene glycol, are also useful to facilitate the solubilization of some perfume ingredients in the composition.

The spray composition may include up to about 5 wt. %, alternatively about 0.1 wt.% to about 2 wt.%, of a malodor counteractant.

*Adjuvants*

Adjuvants can be added to the freshening composition herein for their known purposes. Such adjuvants include, but are not limited to, water soluble metallic salts, including zinc salts, copper salts, and mixtures thereof; antistatic agents; insect and moth repelling agents; colorants; antioxidants; aromatherapy agents and mixtures thereof.

Sprayable Product

The spray composition may be packaged in a spray dispenser to form a sprayable product. The sprayable product may be suitable for use in air and on surfaces.

The sprayable product may be configured to deliver a fine mist. The spray dispenser may be configured in various ways, such as a direct compression-type trigger sprayer, a pre-compression-type trigger sprayer, or an aerosol-type spray dispenser.

An aerosol sprayable product may include a spray composition and a propellant.

Another suitable spray dispenser includes a continuous action sprayer, such as FLAIROSOL™ dispenser from Afa Dispensing Group. The FLAIROSOL™ dispenser includes a bag-in-bag or bag-in-can container with a pre-compression spray engine, and aerosol-like pressurization of the freshening composition without a propellant.

The spray dispenser may be capable of withstanding internal pressure in the range of about 20 p.s.i.g. to about 140 psig, alternatively about 80 to about 130 p.s.i.g.

The total composition output and the spray droplet/particle size distribution may be selected to support the particulate removal efficacy but avoid a surface wetness problem. Total output is determined by the flow rate of the composition it is released from the spray dispenser. To achieve a spray profile that produces minimal surface wetness, it is desirable to have a low flow rate and small spray droplets.

Flow rate is determined by measuring the rate of composition expelled by a container for any 60 seconds period of use. The flow rate of the composition being released from the spray dispenser may be from about 0.0001 grams/second to about 2.5 grams/second. Alternatively, the flow rate may be from about 0.001 grams/second to about 1.8 grams/second, or about 0.01 grams/second to about 1.6 grams/second.

The mean particle size of the spray droplets may be in the range of from about 10 $\square$m to about 100 $\square$m, alternatively from about 20 $\square$m to about 60 $\square$ m. At least some of the spray droplets are sufficiently small in size to be suspended in the air for at least about 10 minutes, and in some cases, for at least about 15 minutes, or at least about 30 minutes.

Small particles can be efficiently created when the spray is dispensed in a wide cone angle. For a given nozzle component and delivery tube, cone angles can be modified by varying the insertion depth of the nozzle in the delivery tube. The cone angle may be greater than about 20 degrees, or greater than about 30 degrees, or greater than about 35 degrees, or greater than about 40 degrees, or greater than about 50 degrees.

The spray dispenser may be configured to spray the composition at an angle that is between an angle that is parallel to the base of the container and an angle that is perpendicular thereto. The desired size of spray droplets can be delivered by other types of spray dispensers that are capable of being set to provide a narrow range of droplet size. Such other spray dispensers include, but are not limited to: foggers, ultrasonic nebulizers, electrostatic sprayers, and spinning disk sprayers.

The freshening composition may be delivered from the spray dispenser which includes delivery components including but not limited to a valve to control flow and to seal the freshening composition within the spray dispenser, a button actuator and a nozzle for dispensing the freshening composition to the environment.

*Propellant*

The sprayable product may include a propellant. Various propellants may be used, including condensable, liquefied gas propellants, such as hydrocarbon (e.g., dimethyl ether, "DME", hydrofluoro olefin "HFO") propellants, hydrofluorocarbon ("HFC") propellants, and compressed gas propellants.

Compressed gas propellants include air, CO2, N20, N2, and the like.

Propellants listed in the U.S. Federal Register 49 C.F.R. §1.73.115, Class 2, Division 2.2 are considered acceptable. The propellant may particularly comprise a trans-1,3,3,3-tetrafluoroprop-1-ene, and optionally a CAS number 1645-83-6 gas. Such propellants provide the benefit that they are not flammable, although the freshening compositions are not limited to inflammable propellants. One such propellant is commercially available from Honeywell International of Morristown, New Jersey under the trade name HFO-1234ze or GWP-6.

By "condensable", it is meant that the propellant transforms from a gaseous state of matter to a liquid state of matter in the spray dispenser and under the pressures encountered in use. Generally, the highest pressure occurs after the spray dispenser is charged with a freshening composition but before that first dispensing of that freshening composition by the user. A condensable propellant provides the benefit of a flatter depressurization curve as the freshening composition is depleted during usage.

The weight ratio of spray composition to propellant present in the sprayable product may be in the range of about 30:70 to about 70:30, alternatively about 40:60 to about 60:40.

The propellant may be a liquefied gas propellant comprising a mixture of propane, n-butane and isobutane having a propellant pressure of the region of 40 psig at 70° F. (2.72 atm at 294K). "Propellant pressure" refers to the approximate vapor pressure of the propellant, as opposed to "can pressure," which refers to the gauge pressure contained within the container of a full aerosol device. A propellant may include a hydrocarbon propellant selected from the group consisting of: n-butane, i-butane, propane and DME, and combinations thereof. A hydrocarbon propellant may include a mixture of n-butane, i-butane and propane collectively known by the trade reference "Butane X", wherein "X" is a number referring to the partial pressure of the mixture in psig at 70° F., with especially preferred examples being Butane 31, Butane 46 and Butane 70.

An Example Spray Composition A is shown below:

| Ingredient | % by Weight | Purpose |
|---|---|---|
| Ethanol | 30-60% | Solvent |
| Butane | 30-60% | Propellant |
| Propane | 10-15% | Propellant |
| Isobutane | 10-15% | Propellant |
| Methanol | 0-2.5% | Solvent |
| Free perfume composition | 0-3.0% | Instant fragrance |
| Perfume-cyclodextrin complex | 0-3.0% | Long-lasting fragrance |
| Malodor counteractant | 0-2.0% | Malodor control |

Example Spray Composition A may leave less wetness on surfaces where it is sprayed than water-based compositions. Example Spray Composition A may also provide long-lasting release of perfume. Example Spray Composition A may also give a triggered release of perfume when the treated surface is exposed to water.

Another exemplary spray composition, Example Spray Composition B, is shown below:

| Ingredient | % by Weight | Purpose |
|---|---|---|
| Isopar M | 30-60% | Solvent |
| Butane | 30-60% | Propellant |
| HFC152a, HFC 134a, HFC227ea, or HFC 236fa (hydro fluorocarbon propellants) | 10-30% | Propellant |

| | | | |
|---|---|---|---|
| Free perfume composition | 0-3.0% | Instant fragrance |
| Perfume-cyclodextrin complex | 0-3.0% | Long-lasting fragrance |
| Malodor counteractant | 0-2.0% | Malodor control |

Spray Composition B may leave less wetness on surfaces where it is sprayed than water-based compositions. Example Spray Composition B may also provide long-lasting release of perfume. Example Spray Composition B may also give a triggered release of perfume when the treated surface is exposed to water, and exhibit a generally consistent spray performance throughout the lifetime of the can.

Method of Use

The sprayable product may be used to treat a surface. A method of using the sprayable product may include spraying a surface with a spray composition and exposing the surface to water. The step of exposing the surface to water may include cleaning the surface with a substrate or by contacting the surface with skin or other sources of water. The treatment may include freshening the surface and/or removing malodors from the surface. The surface may include fabrics, clothing, blankets, curtains, pillows, furniture, carpet, countertops, and the like. The substrate may include a disposable substrate such as paper towel or a dry non-woven wipe. The substrate may also include reusable cloths, towels, or the like. The substrate may be pre-moistened with water or may be wetted with water before cleaning the surface.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A spray composition comprising:
   0.01 wt.% to 3.0 wt.% perfume-cyclodextrin complex, by weight of the overall composition, the perfume-cyclodextrin complex comprising:

   perfume raw materials, wherein 50% or more, by weight of the perfume raw materials have: a cyclodextrin complex stability constant (log k) of less than 3.0, a ClogP of 2.5 or less; and a weight average molecular weight of about 200 Daltons or less; and
   cyclodextrin;
   about 30 wt.% to about 80 wt.% solvent, by weight of the overall composition;
   0.01 wt.% to 3.0 wt. % free perfume composition, by weight of the overall composition; and
   less than 10 wt. % water, by weight of the overall composition.

2. The spray composition according to claim 1, wherein the solvent is selected from the group consisting of: monohydric alcohols, polyhydric alcohols, hydrocarbons, and combinations thereof.

3. The spray composition according to any of the preceding claims further comprising a malodor counteractant.

4. The spray composition according to any of the preceding claims, wherein the cyclodextrin complex stability constant (log k) is from -2.0 to 2.5.

5. The spray composition according to any of the preceding claims, wherein 50% to about 100%, by weight of the perfume-cyclodextrin complex, of the perfume raw materials have: a complex stability constant of 3.0 or less, a ClogP of 2.5 or less; and a weight average molecular weight of about 200 Daltons or less.

6. The spray composition according to any of the preceding claims, wherein the perfume raw materials have a weight average molecular weight of about 180 Daltons or less.

7. A sprayable product comprising:

   a spray dispenser;
   a spray composition comprising:
   a perfume-cyclodextrin complex, by weight of the total composition, the perfume-cyclodextrin complex comprising:

   perfume raw materials, wherein 50% or more, by weight of the perfume raw materials have: a cyclodextrin complex stability constant (log k) of less than 3.0, a ClogP of 2.5 or less; and a weight average molecular weight of about 200 Daltons or less; and

cyclodextrin;

a solvent; and

less than 10 wt. % water, by weight of the total composition; and

a propellant.

8. The sprayable product according to claim 7, wherein the propellant is selected from the group consisting of: butane, propane, or isobutene as propellant, and combinations thereof.

9. The sprayable product according to claim 7 or claim 8, wherein the propellant is selected from the group consisting of: nitrogen, carbon dioxide, air, and combinations thereof.

10. The sprayable product according to any of claims 7 through 9, wherein the weight ratio of spray composition to propellant is about 30:70 to 70:30.

11. The sprayable product according to any of claims 7 through 10, wherein the perfume-cyclodextrin complex is present at a level of 0.01 wt.% to 3.0 wt.%, by weight of the total composition.

12. The sprayable product according to any of claims 7 through 11 further comprising 0.01 wt.% to 2.0 wt. % free perfume composition, by weight of the total composition.

13. The sprayable product according to any of claims 7 through 12, wherein the solvent is present at a level of about 20 wt.% to about 80 wt.% by weight of the spray composition.

14. The sprayable product according to any of claims 7 through 13, wherein the solvent is selected from the group consisting of: monohydric alcohols, polyhydric alcohols, hydrocarbons, and combinations thereof.

15. A method of treating a surface, the method comprising the steps of:

spraying a surface with a spray composition, the spray composition comprising:

a perfume-cyclodextrin complex, by weight of the total composition, the perfume-cyclodextrin complex comprising:

perfume raw materials, wherein 50% or more, by weight of the perfume raw materials have: a cyclodextrin complex stability constant (log k) of less than 3.0, a ClogP of 2.5 or less; and a weight average molecular weight of about 200 Daltons or less; and

cyclodextrin;

a solvent; and

less than 10 wt. % water, by weight of the total composition; and

exposing the surface to water.

**Patentansprüche**

1. Sprühzusammensetzung, umfassend:

zu 0,01 Gew.-% bis 3,0 Gew.-% Duftstoff-Cyclodextrin-Komplex, bezogen auf das Gewicht der Gesamtzusammensetzung, wobei der Duftstoff-Cyclodextrin-Komplex umfasst:

Duftstoffrohmaterialien, wobei 50 Gew.-% oder mehr, bezogen auf das Gewicht der Duftstoffrohmaterialien, aufweisen: eine Cyclodextrin-Komplex-Stabilitätskonstante (log k) von weniger als 3,0, einen ClogP von 2,5 oder weniger; und ein gewichtsgemitteltes Molekulargewicht von etwa 200 Dalton oder weniger; und

Cyclodextrin;

zu etwa 30 Gew.-% bis etwa 80 Gew.-% Lösungsmittel, bezogen auf das Gewicht der Gesamtzusammensetzung;

zu 0,01 Gew.-% bis 3,0 Gew.-% freie Duftstoffzusammensetzung, bezogen auf das Gewicht der Gesamtzusammensetzung; und

zu weniger als 10 Gew.-% Wasser, bezogen auf das Gewicht der Gesamtzusammensetzung.

2. Sprühzusammensetzung nach Anspruch 1, wobei das Lösungsmittel ausgewählt ist aus der Gruppe bestehend aus: einwertigen Alkoholen, mehrwertigen Alkoholen, Kohlenwasserstoffen und Kombinationen davon.

3. Sprühzusammensetzung nach einem der vorstehenden Ansprüche, ferner umfassend einen Geruchsneutralisierer.

4. Sprühzusammensetzung nach einem der vorstehenden Ansprüche, wobei die Cyclodextrin-Komplex-Stabilitätskonstante (log k) von 2,0 bis 2,5 beträgt.

5. Sprühzusammensetzung nach einem der vorstehenden Ansprüche, wobei 50 Gew.-% bis etwa 100 Gew.-%, bezogen auf das Gewicht des Duftstoff-Cyclodextrin-Komplexes, der Duftstoffrohmaterialien aufweisen: eine Komplex-Stabilitätskonstante von 3,0 oder weniger, einen ClogP von 2,5 oder weniger; und ein gewichtsgemitteltes Molekulargewicht von etwa 200 Dalton oder weniger.

6. Sprühzusammensetzung nach einem der vorstehenden Ansprüche, wobei die Duftstoffrohmaterialien ein gewichtsgemitteltes Molekulargewicht von etwa 180 Dalton oder weniger aufweisen.

7. Sprühbares Produkt, umfassend:

   einen Sprühspender;
   eine Sprühzusammensetzung, umfassend:
   einen Duftstoff-Cyclodextrin-Komplex, wobei der Duftstoff-Cyclodextrin-Komplex, bezogen auf das Gewicht der Gesamtzusammensetzung, umfasst:

      Duftstoffrohmaterialien, wobei 50 Gew.-% oder mehr, bezogen auf das Gewicht der Duftstoffrohmaterialien, aufweisen: eine Cyclodextrin-Komplex-Stabilitätskonstante (log k) von weniger als 3,0, einen ClogP von 2,5 oder weniger; und ein gewichtsgemitteltes Molekulargewicht von etwa 200 Dalton oder weniger; und Cyclodextrin;
      ein Lösungsmittel; und
      zu weniger als 10 Gew.-% Wasser, bezogen auf das Gewicht der Gesamtzusammensetzung; und
      ein Treibmittel.

8. Sprühbares Produkt nach Anspruch 7, wobei das Treibmittel ausgewählt ist aus der Gruppe bestehend aus: Butan, Propan oder Isobuten als Treibmittel und Kombinationen davon.

9. Sprühbares Produkt nach Anspruch 7 oder Anspruch 8, wobei das Treibmittel ausgewählt ist aus der Gruppe bestehend aus: Stickstoff, Kohlenstoffdioxid, Luft und Kombinationen davon.

10. Sprühbares Produkt nach einem der Ansprüche 7 bis 9, wobei das Gewichtsverhältnis von Sprühzusammensetzung zu Treibmittel etwa 30:70 bis 70:30 beträgt.

11. Sprühbares Produkt nach einem der Ansprüche 7 bis 10, wobei der Duftstoff-Cyclodextrin-Komplex in einer Menge von 0,01 Gew.-% bis 3,0 Gew.-%, bezogen auf das Gewicht der Gesamtzusammensetzung, vorliegt.

12. Sprühbares Produkt nach einem der Ansprüche 7 bis 11, ferner umfassend zu 0,01 Gew.-% bis 2,0 Gew.-% freie Duftstoffzusammensetzung, bezogen auf das Gewicht der Gesamtzusammensetzung.

13. Sprühbares Produkt nach einem der Ansprüche 7 bis 12, wobei das Lösungsmittel in einer Menge von etwa 20 Gew.- bis etwa 80 Gew. -% der Sprühzusammensetzung vorliegt.

14. Sprühbares Produkt nach einem der Ansprüche 7 bis 13, wobei das Lösungsmittel ausgewählt ist aus der Gruppe bestehend aus: einwertigen Alkoholen, mehrwertigen Alkoholen, Kohlenwasserstoffen und Kombinationen davon.

15. Verfahren zum Behandeln einer Oberfläche, wobei das Verfahren die Schritte umfasst:
    Besprühen einer Oberfläche mit einer Sprühzusammensetzung, wobei die Sprühzusammensetzung umfasst:
    einen Duftstoff-Cyclodextrin-Komplex, wobei der Duftstoff-Cyclodextrin-Komplex, bezogen auf das Gewicht der Gesamtzusammensetzung, umfasst:

    Duftstoffrohmaterialien, wobei 50 Gew.-% oder mehr, bezogen auf das Gewicht der Duftstoffrohmaterialien, aufweisen: eine Cyclodextrin-Komplex-Stabilitätskonstante (log k) von weniger als 3,0, einen ClogP von 2,5 oder weniger; und ein gewichtsgemitteltes Molekulargewicht von etwa 200 Dalton oder weniger; und Cyclodextrin;

ein Lösungsmittel; und

zu weniger als 10 Gew.-% Wasser, bezogen auf das Gewicht der Gesamtzusammensetzung; und

Aussetzen der Oberfläche gegenüber Wasser.

## Revendications

1. Composition d'aérosol comprenant :

0,01 % en poids à 3,0 % en poids de complexe parfum/cyclodextrine, en poids de la composition globale, le complexe parfum/cyclodextrine comprenant :

des matières premières de parfum, dans laquelle 50 % ou plus, en poids des matières premières de parfum ont : une constante de stabilité du complexe de cyclodextrine (log k) inférieure à 3,0, un ClogP de 2,5 ou moins ; et une masse moléculaire moyenne en poids d'environ 200 Daltons ou moins ; et

de la cyclodextrine ;

environ 30 % en poids à environ 80 % en poids de solvant, en poids de la composition globale ;

0,01 % en poids à 3,0 % en poids de composition parfumée libre, en poids de la composition globale ; et

moins de 10 % en poids d'eau, en poids de la composition globale.

2. Composition d'aérosol selon la revendication 1, dans laquelle le solvant est choisi dans le groupe constitué de : alcools monohydriques, alcools polyhydriques, hydrocarbures, et des combinaisons de ceux-ci.

3. Composition d'aérosol selon l'une quelconque des revendications précédentes comprenant en outre un agent neutralisant les mauvaises odeurs.

4. Composition d'aérosol selon l'une quelconque des revendications précédentes, dans laquelle la constante de stabilité du complexe de cyclodextrine (log k) va de 2,0 à 2,5.

5. Composition d'aérosol selon l'une quelconque des revendications précédentes, dans laquelle 50 % à environ 100 %, en poids du complexe parfum/cyclodextrine, des matières premières de parfum ont : une constante de stabilité du complexe de 3,0 ou moins, un ClogP de 2,5 ou moins ; et une masse moléculaire moyenne en poids d'environ 200 Daltons ou moins.

6. Composition d'aérosol selon l'une quelconque des revendications précédentes, dans laquelle les matières premières de parfum ont une masse moléculaire moyenne en poids d'environ 180 Daltons ou moins.

7. Produit vaporisable comprenant :

un atomiseur ;

une composition d'aérosol comprenant :

un complexe parfum/cyclodextrine, en poids de la composition totale, le complexe parfum/cyclodextrine comprenant :

des matières premières de parfum, dans lequel 50 % ou plus, en poids des matières premières de parfum ont : une constante de stabilité du complexe de cyclodextrine (log k) inférieure à 3,0, un ClogP de 2,5 ou moins ; et une masse moléculaire moyenne en poids d'environ 200 Daltons ou moins ; et

de la cyclodextrine ;

un solvant ; et

moins de 10 % en poids d'eau, en poids de la composition totale ; et

un propulseur.

8. Produit vaporisable selon la revendication 7, dans lequel le propulseur est choisi parmi le groupe constitué de : butane, propane, ou isobutène en guise de propulseur, et des combinaisons de ceux-ci.

9. Produit vaporisable selon la revendication 7 ou la revendication 8, dans lequel le propulseur est choisi parmi le groupe constitué de : azote, dioxyde de carbone, air, et des combinaisons de ceux-ci.

10. Produit vaporisable selon l'une quelconque des revendications 7 à 9, dans lequel le rapport massique de composition d'aérosol à propulseur est d'environ 30:70 à 70:30.

11. Produit vaporisable selon l'une quelconque des revendications 7 à 10, dans lequel le complexe parfum/cyclodextrine est présent à un niveau de 0,01 % en poids à 3,0 % en poids, en poids de la composition totale.

12. Produit vaporisable selon l'une quelconque des revendications 7 à 11 comprenant en outre 0,01 % en poids à 2,0 % en poids de composition parfumée libre, en poids de la composition totale.

13. Produit vaporisable selon l'une quelconque des revendications 7 à 12, dans lequel le solvant est présent à un niveau d'environ 20 % en poids à environ 80 % en poids en poids de la composition d'aérosol.

14. Produit vaporisable selon l'une quelconque des revendications 7 à 13, dans lequel le solvant est choisi parmi le groupe constitué de : alcools monohydriques, alcools polyhydriques, hydrocarbures, et des combinaisons de ceux-ci.

15. Procédé de traitement d'une surface, le procédé comprenant les étapes consistant à :
pulvériser une surface avec une composition d'aérosol, la composition d'aérosol comprenant :
un complexe parfum/cyclodextrine, en poids de la composition totale, le complexe parfum/cyclodextrine comprenant :

des matières premières de parfum, dans lequel 50 % ou plus, en poids des matières premières de parfum ont :
une constante de stabilité du complexe de cyclodextrine (log k) inférieure à 3,0, un ClogP de 2,5 ou moins ; et
une masse moléculaire moyenne en poids d'environ 200 Daltons ou moins ; et
de la cyclodextrine ;
un solvant ; et
moins de 10 % en poids d'eau, en poids de la composition totale ; et
exposer la surface à de l'eau.

FIG. 1

EP 3 290 087 B1

FIG. 2

FIG. 3

EP 3 290 087 B1

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006138726 A

**Non-patent literature cited in the description**

- **REKHARSKY ; INOUE.** Complexation Thermodynamics of Cyclodextrins. *Chemical Review,* 1998, vol. 98, 1875-1917

- **VAN GEMERT, L.J.** *Odour Thresholds (Compilations of Odour Threshold Values in Air, Water and Other Media; Oliemans Punter & Partners;,* 2011
- *Atomic Weights of the Elements,* 2005
- *United States Pharmacopeia,* 01 August 2006